# EUROPEAN PATENT APPLICATION

(11) **EP 1 930 438 A1**
(43) Date of publication of application: **11.06.2008**
(21) Application number: 06077192.0
(22) Date of filing: 07.12.2006
(51) Int. Cl.: C12N 15/12, C07K 14/47, C12Q 1/68, A61K 39/00

(54) **Transcription factor for killer cell activation, differentiation and uses thereof**

(71) Applicant: Academisch Medisch Centrum bij de Universiteit van Amsterdam, 1105 AZ Amsterdam (NL)
(72) Inventor: The designation of the inventor has not yet been filed
(74) Representative: van Loon, C.J.J.

(57) **Abstract**

The present invention provides methods for altering activation and/or differentiation of Killer cells comprising altering the expression of a Homologue Of Blimp-1 In T-cells (Hobit) species in said Killer-cells. The invention further provides methods and means for increasing or decreasing expression of a Hobit species in Killer cells, and provides isolated and/or recombinant nucleic acid and uses of said isolated and/or recombinant nucleic acid.

## Description

The invention relates to the field of immunology. The invention in particular relates to means and methods for modulating an immune response in an individual.

The hemopoietic system is a complex system containing a large variety of different cell types. These cells include the very abundant and differentiated red blood cells, B- and T-cells, monocytes and macrophages. These differentiated cells often have a limited time span and need to be replenished continuously. This is achieved through a cascade of increasingly more primitive cells that have self-renewal capacity and differentiation capacity. In general it can be said that the more primitive the precursor cell the more self-renewal capacity the cell will have and the more different cell types the precursor can generate. The top of the cascade is formed by the so-called pluripotent stem cell that has extensive self-renewal capacity and the capacity to generate every differentiated hemopoietic cell of the adult system. This stem cell can, through differentiation into a series of more committed progenitor cells, provide the hemopoietic system with the various types of differentiated cells. Stem cells and progenitor cells are preferred examples of precursor cells as used herein.

Many different markers have been identified with which the various types of hemopoietic cells can be distinguished from one another. Also many of the growth factors and other external signals that direct the process of differentiation have been identified. However, although much is known about the various differentiated cells and their precursors; surprisingly little is known about the nuclear factors that are the mediators of the differentiation process.

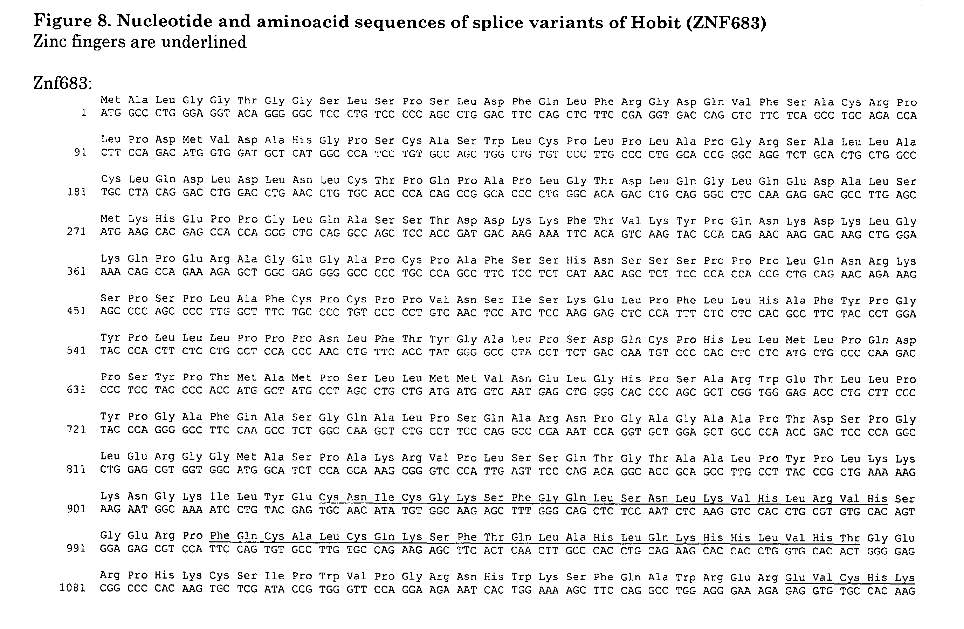

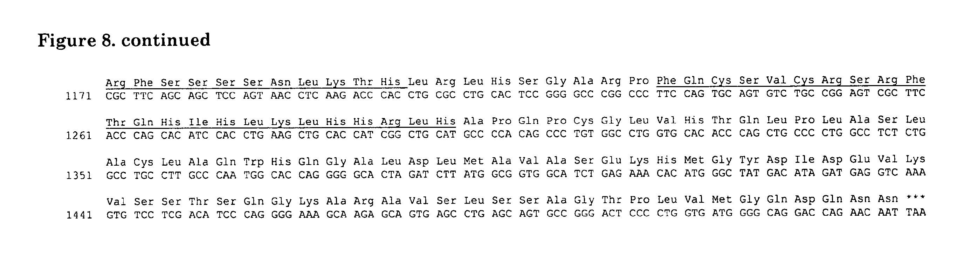

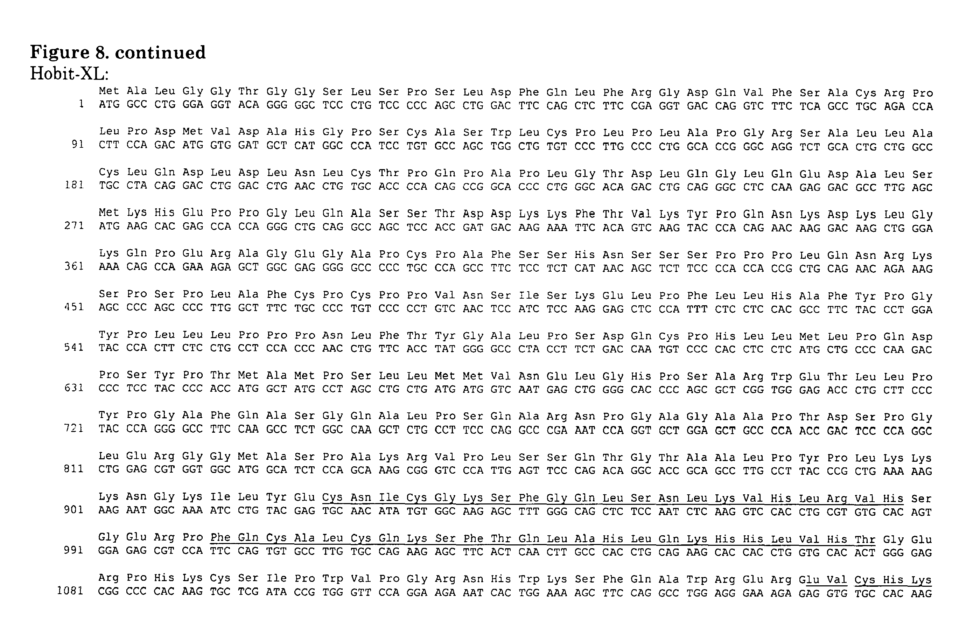

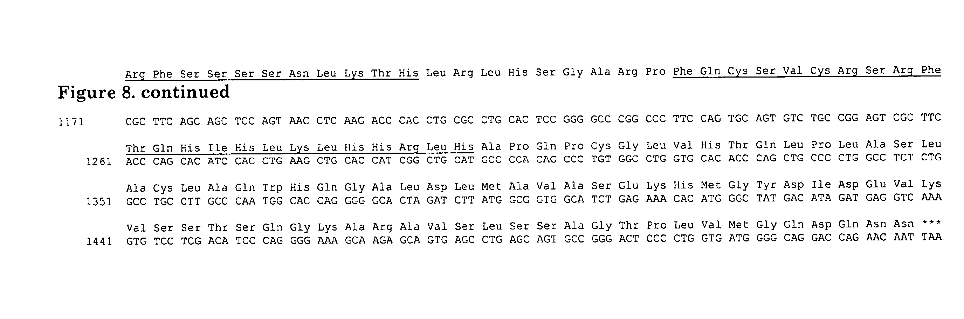

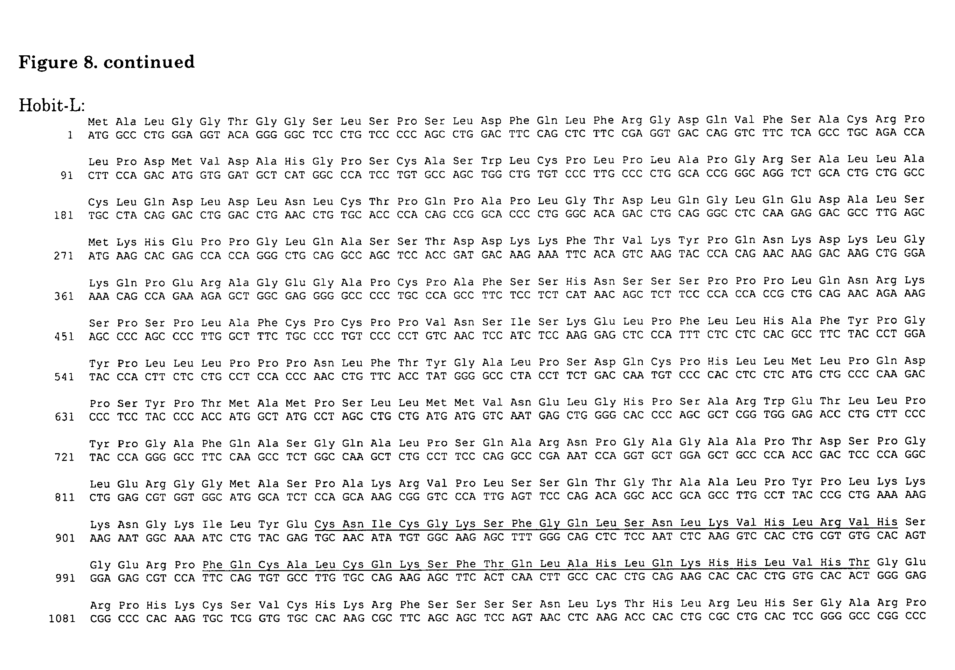

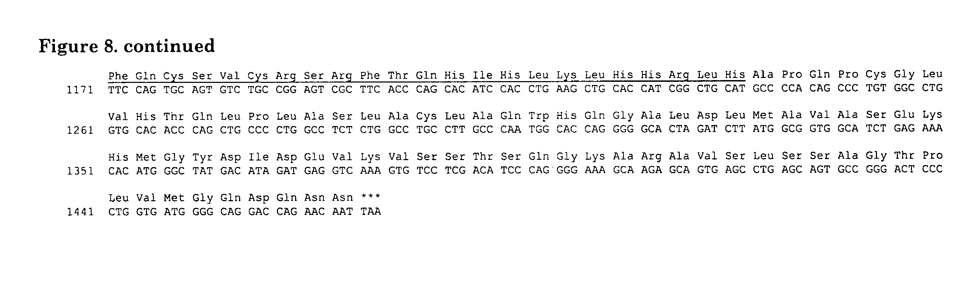

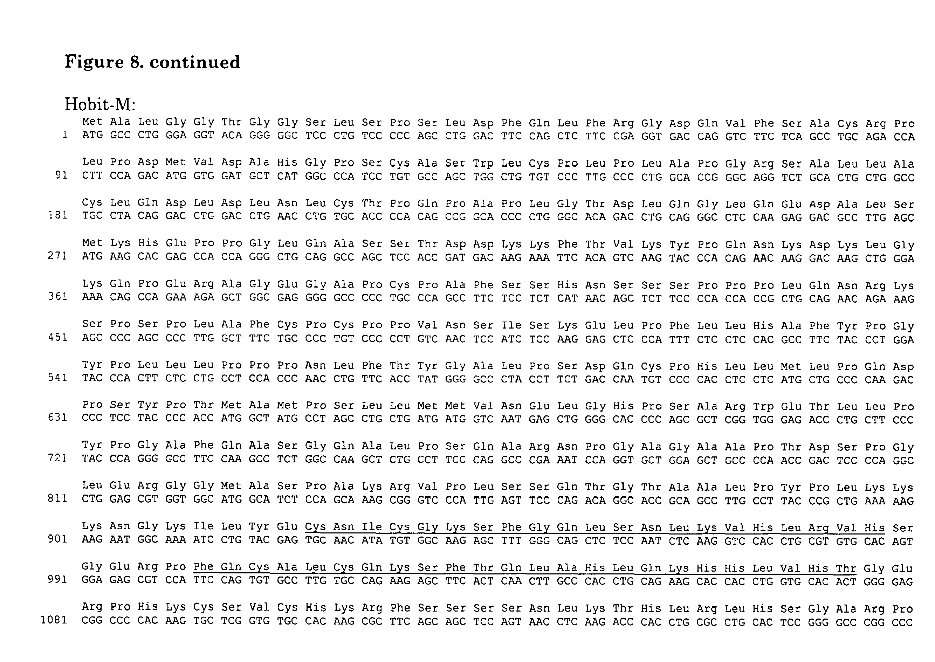

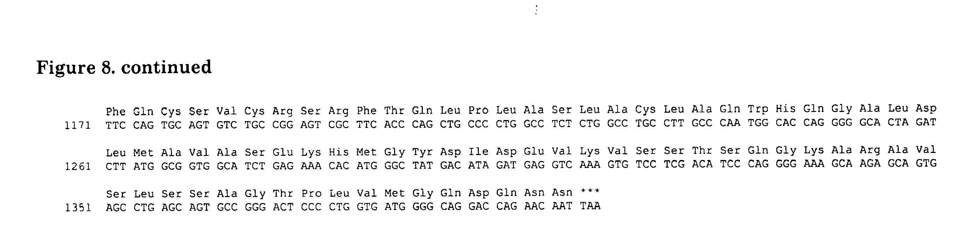

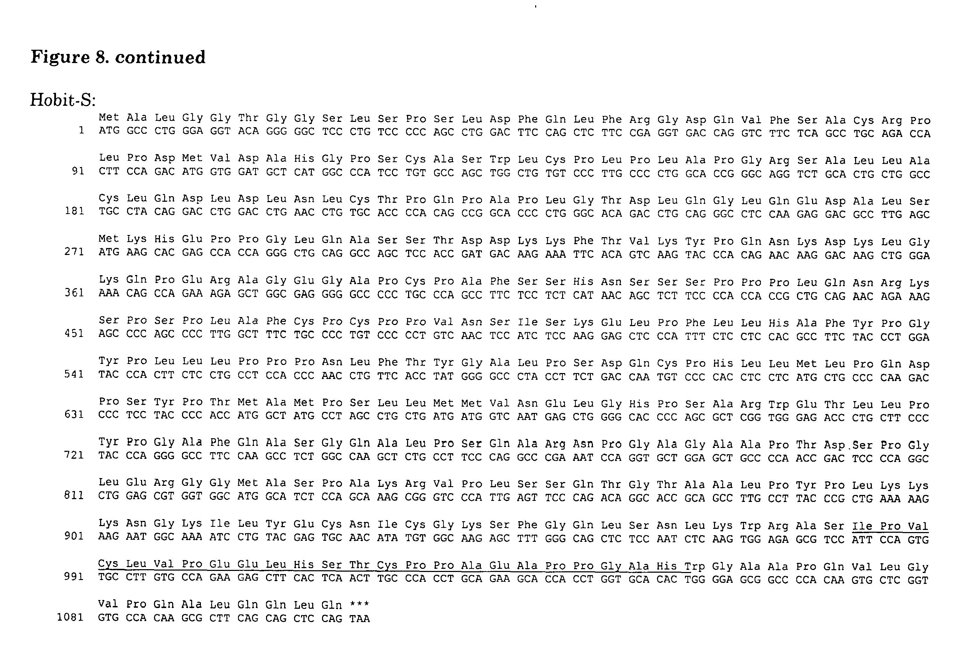

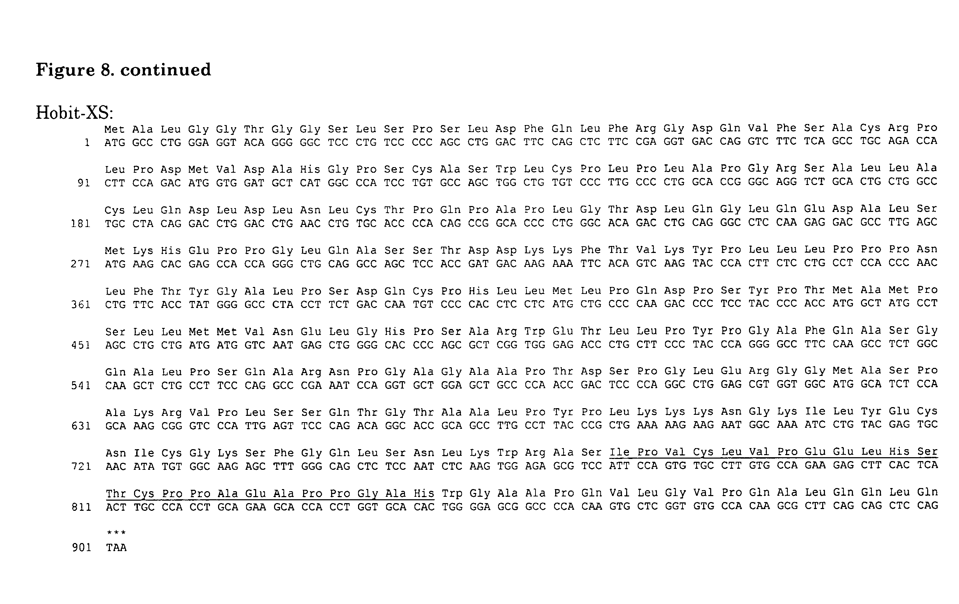

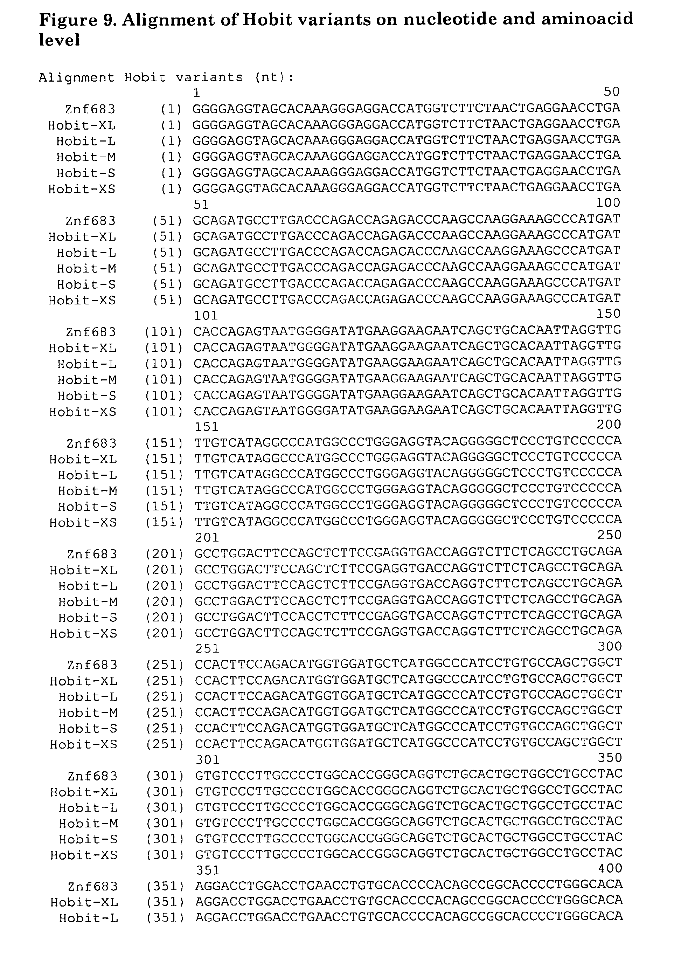

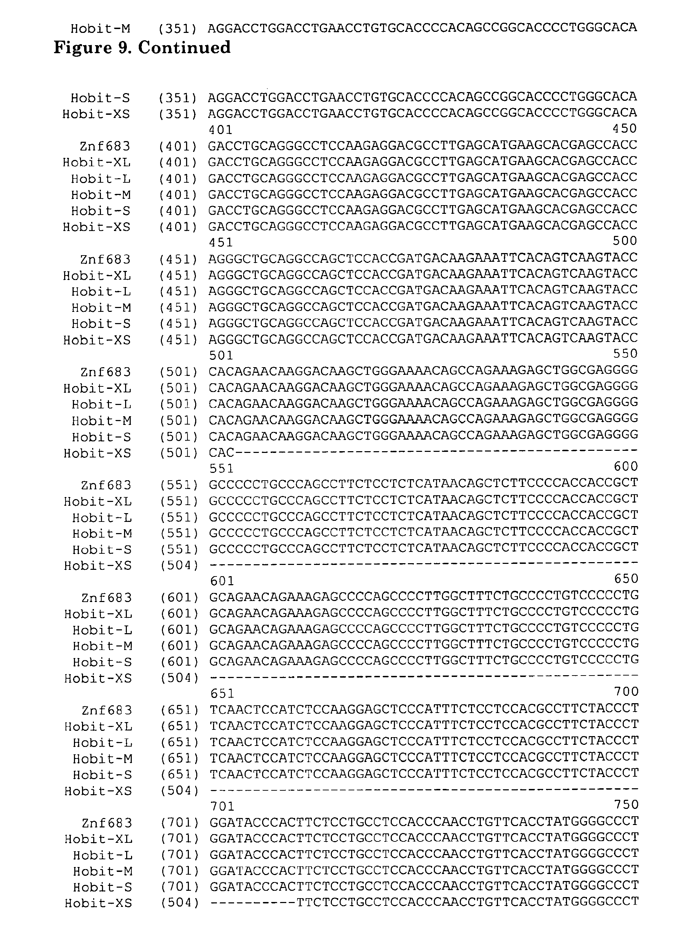

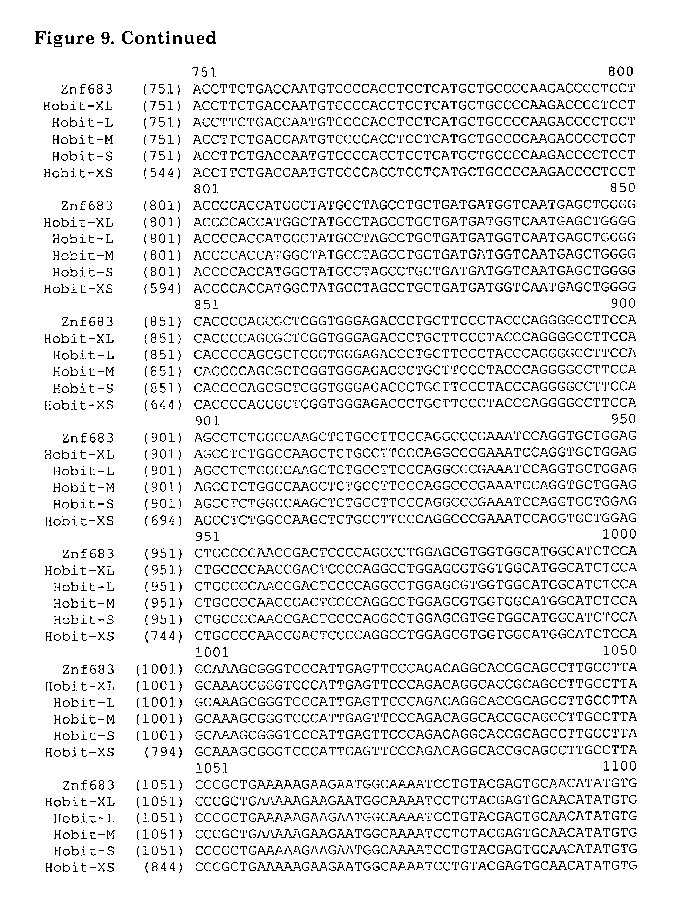

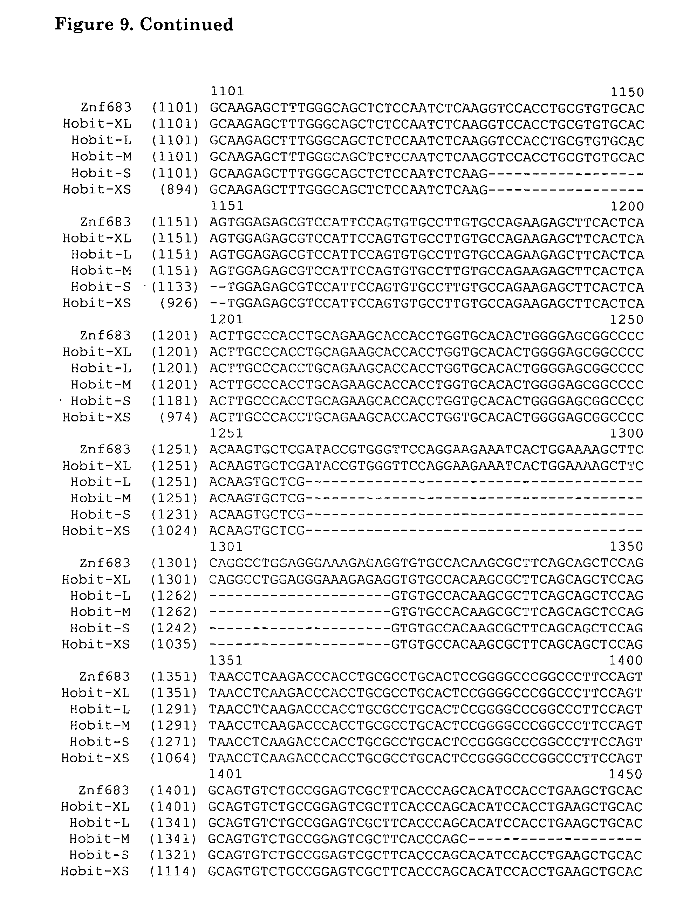

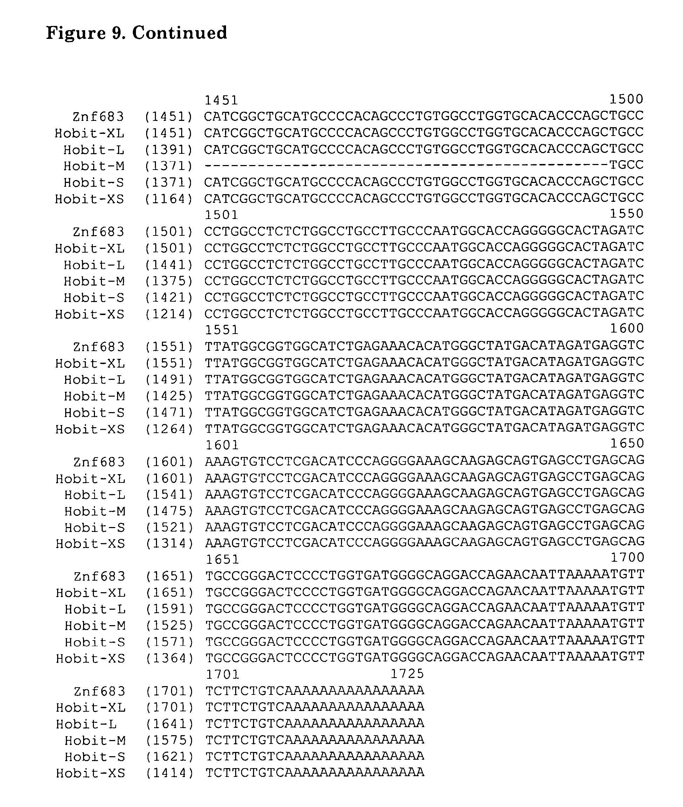

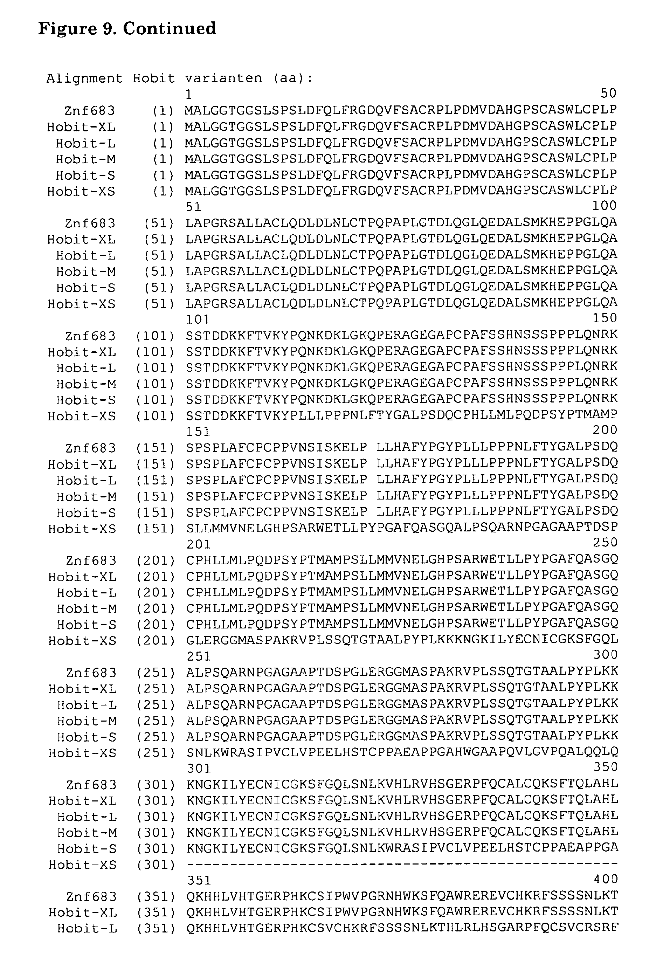

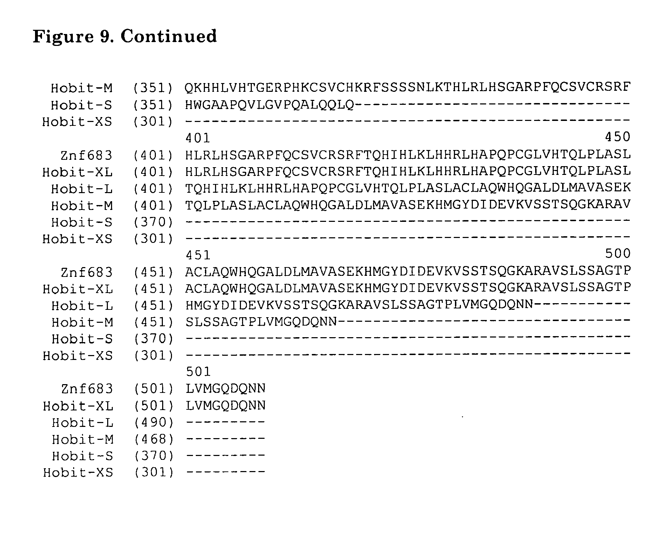

An exception to this rule is perhaps red cell differentiation. Here several different transcription factors have been identified that at least play a role in the expression of the globin gene during development and during differentiation of the committed red blood cell progenitors. In the B-cell lineage, a transcription factor called Blimp-1 has been identified. This factor is involved in the maturation of B-cells into plasma cells. In the present invention a Homologue Of Blimp-1 In T-cells (Hobit) has been identified. Analysis of mRNA in Hobit expressing cells revealed the presence of a number differently spliced variants (further referred to as Hobit species). The amino acid sequence of the different splice variants is given in figures 8 or 9.

Expression of Hobit species is induced in response to activation and/or differentiation of Killer-cells and/or when an infection needs to be combated. Vise versa, Hobit expression in resting Killer cells and/or naive T-cells activates and/or differentiates these cells.

All Killer cells ultimately derive from haematopoietic stem cells in the bone marrow. Hematopoietic progenitors derived from haematopoietic stem cells populate the thymus and expand by cell division to generate a large population of immature "thymocytes". The earliest thymocytes express neither CD4 nor CD8, and are therefore classed as double negative (CD4-CD8-) cells. As they progress through their°development they become double positive thymocytes (CD4⁺CD8⁺), and finally mature to single positive (CD4⁺CD8⁻ or CD4-CD8⁺) thymocytes that are then released from the thymus to peripheral tissues.

About 98% of thymocytes die during the development processes in the thymus by failing either positive selection or negative selection, while the other 2% survive and leave the thymus to become mature immunocompetent T cells. In bone marrow transplantations it has been observed that stem cells from the graft repopulate also the T-cell lineage. As bone marrow transplantation is often performed in adults that no longer have a fully developed thymus, it is thought that replenishment of the T-cell lineage and education of the T-cell such that they do not recognize the "self"-antigens of the host continues throughout the life of an adult, albeit at a low level in normal situations. Macrophages are part of the myeloid lineage and also originate from the pluripotent hemopoietic stem cell. They do not pass through the thymus. NK cells also originate from the pluripotent hemopoietic stem cell and are thought to derive from a lymphoid progenitor they share with B-cells and T-cells.

T-cell activation varies slightly between the different types of T-cells; however, the following two-signal model is generally true for most: The interaction between TCR molecules and specific MHC/antigen complexes on antigen presenting cells (APCs) delivers signal 1 into the T cell. Co-stimulatory interactions between CD28 molecules on the T cell and B7 molecules on the APC deliver signal 2, activating the T cell. Without co-stimulation a T cell will become functionally inert (anergic). T cell activation can be followed by measuring proliferation, usually by incorporation of radioactive thymidine, or secretion of IL-2, an event which is dependent on co-stimulation.

A naive T cell is considered mature, but is distinguished from activated T cells or memory T cells, as it is thought to not have yet encountered cognate antigen in the periphery. Naive T cells are commonly characterized by the surface co-expression of the surface markers L-selectin (CD62L) and CD45RA; the absence of the activation markers CD38 and CD69; and the absence of memory markers, such as the CD450 isoform. In the naive state, T cells are thought to be quiescent and non-dividing, requiring the common-gamma chain cytokines IL7 and IL15 for homeostatic survival. Naive T cells are able to respond to novel pathogens that the immune system has not yet encountered. Recognition by a naive T cell clone of its cognate antigen results in the initiation of an acquired immune response. In the ensuing response, the T cell acquires an activated phenotype (IL7R-CD38+CD45R0+), and may further differentiate into a memory T cell (CD38-CD45R0+).

Memory T cells are a specific type of infection-fighting T cell (also known as a T lymphocyte) that can recognize antigens such as bacterial, viral and tumour specific antigens, that were encountered during a prior infection or vaccination. At a second encounter with the antigen, memory T cells can reproduce to mount a faster and stronger immune response than the first time the immune system responded to the antigen. There are several distinct populations of memory T cells, based on phenotypic and functional properties. Memory T cells can be recognized by the differential expression of certain molecules. Central memory T_{CM} cells express L-selectin and the chemokine receptor CCR7, they secrete IL-2, but little IFNγ or IL-4. Effector memory TEM cells, however, do not express L-selectin or CCR7 but produce effector cytokines like IFNγ and IL-4. Antigen-specific memory T cells against viruses or other microbial molecules can be found in both T_{CM} and T_{EM} subsets. Although most information is currently based on observations in the Cytotoxic T cells (CD8-positive) subset, similar populations appear to exist for both the Helper T cells (CD4-positive) and the cytotoxic T cells.

Within the human cytotoxic T cell population, three distinct subpopulations have now been described: central memory (T_{CM}), effector memory (T_{EM}), and effector memory RA (T_{EMRA}) T cells, giving two highly related effector memory sub-types. The T_{CM} cells are thought to represent memory stem cells. T_{EM} and T_{EMR}A cells strongly express genes for molecules essential to the cytotoxic function of CD8 T cells, whereas T_{CM} display a capacity for self-renewal due to high levels of phosphorylation of the transcription factor known as STAT5.

Natural killer (NK) cells are a form of cytotoxic lymphocyte. Cytotoxic lymphocytes constitute a major component of the innate immune system. Within the context of the innate immune system, NK-cells act to lyse cells in a way that does not require the MHC-presentation of a specific "disease" associated antigen. NK cells were discovered due to their non-MHC-restricted killer activity that disturbed early attempts to generate tumour-specific, MHC-restricted cytotoxic T lymphocytes (CTLs) from mice shortly after the discovery of the phenomenon of MHC restriction. NK cells are large granular lymphocytes that do not express T-cell antigen receptors (TCR) or Pan T marker CD3 or surface immunoglobulins (Ig) B cell receptor but which usually express the surface markers CD16 (FcγRIII) and CD56 in humans, and NK1.1/NK1.2 in certain strains of mice.

With the discovery of activating receptors almost two decades after the discovery of the inhibitory receptors these cells continue to be called by the same name, though "natural" does not mean the same any more. However the term "natural killer" continues to be justified by:
NK cells are cytotoxic; small granules in their cytoplasm contain special proteins such as perforin and proteases known as granzymes. Upon release in close proximity to a cell slated for killing, perforin forms pores in the cell membrane of the target cell through which the granzymes and associated molecules can enter, inducing apoptosis. The distinction between apoptosis and cell lysis is important in immunology- lysing a virus-infected cell would only release the virions, whereas apoptosis leads to destruction of the virus inside.
NK cells are activated in response to type 1 interferons or macrophage-derived cytokines. They serve to contain among others viral infections while the adaptive immune response is generating antigen-specific cytotoxic T cells that can clear the infection. Patients deficient in NK cells prove to be highly susceptible to early phases of herpes virus infection. NK cells are currently also investigated as killer cells for the destruction of tumours.

In order for NK cells to defend the body against viruses and other pathogens, they require mechanisms which enable the determination of whether a cell is infected or not. The exact mechanisms remain the subject of current investigation, but recognition of an "altered self" state is thought to be involved. This "altered self" state also seems to be present on at least some types of tumour cells.
To control the cytotoxic activity, NK cells possess two types of surface receptors: "activating receptors" and "inhibitory receptors". Most of these receptors are not unique to NK cells and can be present in other T cell subsets as well.

These inhibitory receptors recognize MHC class I alleles, which could explain why NK cells kill cells possessing low levels of MHC class I molecules. NK cell receptor types (with inhibitory as well as some activating members) are differentiated by structure:
CD94 : NKG2 (heterodimers) - a C-type lectin family receptor, conserved in both rodents and primates and identifies non-classical (also non-polymorphic) MHC I molecules like HLA E. Though indirect, this is a way to survey the levels of classical (polymorphic) HLA molecules, however, because expression of HLA-E at the cell surface is dependent upon the presence of classical MHC class I leader peptides.
Ly49 (homodimers) - a relatively ancient, C-type lectin family receptor; are of multigenic presence in mice, while humans have only one pseudogenic Ly49; the receptor for classical (polymorphic) MHC I molecules.
KIR (Killer cell Immunoglobulin-like Receptors) - belong to a multigene family of more recently-evolved Ig-like extracellular domain receptors; are present in non- rodent primates; and are the main receptors for both classical MHC I (HLA A,HLA B,HLA C) and also non-classical HLA G in primates. Some KIRs are specific for certain HLA subtypes.
ILT or LIR (leucocyte inhibitory receptors) - are recently-discovered members of the Ig receptor family. Activation of NK cells is preferably determined by measuring Nkp44 and/or 46.

Macrophages differentiate from monocytes. When a monocyte enters the attacked tissue through the endothelium of a blood vessel (a process known as the leukocyte adhesion cascade), it undergoes a series of changes and becomes a macrophage. Macrophages are attracted to a damaged site by chemical substances through chemotaxis, triggered by a range of different stimuli including damaged cells, pathogens, histamine released by mast cells and basophils, and cytokines released by macrophages already at the site. The life span of a macrophage ranges from months to years. Macrophages are unable to divide and must mature from monocytes produced in the bone marrow. A monocyte is therefore considered to be one of the precursors of a macrophage.

The present invention provides a method for altering activation and/or differentiation of Killer cells comprising altering the expression of a Hobit species in said Killer-cells. In a preferred embodiment of the invention said activation and/or differentiation is elevated through increasing expression of said Hobit species in said Killer-cell. When Killer-cells of a collection of Killer-cells are provided with a Hobit species it allows activation of a resting Killer cell and/or naive T-cell as measured by the expression of at least one activation marker for the specific Killer cell and/or T-cell. Killer-cells that are not fully activated become more strongly activated as measured by the increased expression of said activation marker. Fully activated Killer-cells proceed to a more differentiated effector phenotype as evidenced for instance in T-cells by the strong increase in IFNg secretion. A collection of Killer-cells with a certain overall activation level will become more activated as a result of the provision of said Hobit species. This process can be measured by an increase in the overall expression level of said activation marker. Differentiation of Killer-cells often, but not necessarily always, follows the activation state of the Killer-cells and can be measured using the same activation marker.
In another preferred embodiment said activation and/or differentiation is reduced through decreasing expression of said Hobit species in said Killer-cell. Reduction of activation and/or differentiation is achieved by lowering or decreasing the expression of said Hobit species in an activated and/or differentiated Killer-cell that expresses said Hobit species.

A lowering of the expression can be achieved in a number of different ways. Preferably, but not necessarily, said cell is provided with a nucleic acid that contains an anti-sense sequence for RNA encoding said Hobit species. There are several types of anti-sense strategies that can be used. Non-limiting examples are the provision of a cell with a siRNA, a miRNA, a hairpin RNA or DNA molecule, the use of oligonucleotides that can induce exon-skipping and other strategies. Many of these strategies use analogues of nucleic acid to increase delivery, stability or other factors to improve the efficiency of the reduction of level and/or translation of the target RNA molecule. Such modifications and/or analogues of nucleic acid are in the present invention functional equivalents of a nucleic acid containing an anti-sense sequence. Non-limiting examples of such functional equivalents are LNA, PNA, 2-O-methyl modified oligonucleotides, morpholino's and others. Functional equivalents are for instance also nucleic acids that contain one or more nucleotide analogues. Non-limiting examples of such nucleotide analogues are those that have been developed and that will be developed because of their effect on viral replication. Others such as inosine are developed for a different purpose. A general characteristic of a nucleotide analogue of the present invention is that it can be incorporated into a nucleic acid molecule be it in a synthetic process or in a natural process of nucleic acid synthesis, or in a combination thereof. A reduction of activation and/or differentiation of a Killer-cell is preferably measured by a lowering or reduction in the expression of said activation marker on said cell. A reduction of activation and/or differentiation of a collection of Killer-cells containing activated Killer-cells is preferably measured by measuring a lowering or reduction in the overall expression of said activation marker in said collection of Killer-cells.

Killer-cells can, as mentioned herein above, be replenished from the bone marrow in pre-pubertal individuals and also, albeit to a lesser extend, in post-pubertal individuals. In the context of the present invention it is possible to affect the activation and/or differentiation potential of Killer-cells formed in this process by providing a Killer-cell precursor of said Killer-cell with a nucleic acid for altering the expression said Hobit species in a progeny Killer-cell of said precursor. When provided with a nucleic acid or functional equivalent thereof comprising an anti-sense sequence of said Hobit species it is possible to reduce the potential for activation and/or differentiation of the resulting progeny Killer-cell. When the precursor of said Killer-cell is provided with a nucleic acid for enhancing and/or elevating expression of said Hobit species in the resulting Killer-cell, said Killer-cell becomes activated as a result of the elevated enhanced and/or elevated expression thereof.

In a preferred embodiment of the invention said nucleic acid is provided to a naive T-cell, an NK cell or an antigen specific T cell. An antigen specific T-cell is preferably specific for a tumour and/or a virus. An antigen specific T-cell provided with a nucleic acid encoding a Hobit species of the invention will become activated and/or more differentiated when provided with said nucleic acid for enhancing and/or elevating expression of said Hobit species in said antigen specific T-cell. Similarly, a collection of antigen specific T-cells will become more activated and/or more differentiated. These cells are preferably used for adoptive therapy. A naive T-cell provided with a nucleic acid encoding a Hobit species of the invention will become activated and/or more differentiated when provided with said nucleic acid for enhancing and/or elevating expression of said Hobit species in said naive T-cell. Similarly, a collection of naive T-cells will become more activated and/or more differentiated. A naive T-cell provided with a nucleic acid and/or functional equivalent thereof, for lowering and/or reducing expression of said Hobit species is less inclined to become activated and/or differentiated when induced. Similarly, a collection of naive T-cells provided with said a nucleic acid and/or functional equivalent thereof, for lowering and/or reducing expression of said Hobit species is less inclined to become activated and/or differentiated when induced. The activation state of said naive T-cell and/or said collection of naive T-cells, prior to or following the provision of said nucleic acid can be measured as described herein above.

The present invention is preferably applied in humans and/or human cells in vitro. However, other animals a such as but not limited to other mammals and even birds have a corresponding homologue of human Hobit. The present invention can therefore also be applied to (cells of) said other species. In a preferred embodiment said Killer-cell or precursor thereof is a human Killer-cell or human precursor thereof.

The mentioned cells are preferably in vitro provided with a means for altering expression of said Hobit species. Preferably said expression is increased in cells in vitro. A preferred application of the present invention is the improvement of the immune capability of a graft. Grafting of autologous, matched and mismatched cells has become a standard practise in the clinic for a variety of different diseases. Many of the grafts that are transplanted contain immune cells. These cells are often transplanted for the purpose of fortifying the immune system of the recipient. In many cases this is done to combat tumours in the recipient. Many of these transplantations show some effect of the transplanted immune cells on the tumour cells. In some cases the effect is indeed very impressive. However, often also the effect is transient and insufficient to remove a significant number of the tumour cells for the recipient. The transplanted immune cells are apparently often not sufficiently activated/differentiated to combat the tumour efficiently. In the art various strategies are pursued to increase the tumour killing potential of the grafted immune cells. In the present invention it was found that the activation and/or differentiation of immune cells in a graft is elevated and/or enhanced upon increasing expression of a Hobit species in immune cells and/or precursors thereof of said graft.
In one embodiment a method of the invention is used for enhancing a Killer-cell response of said Killer-cells or progeny thereof. In a preferred embodiment a method of the invention is used for enhancing an antiviral response and/or an anti-tumour response of said Killer-cells, preferably antigen-specific T-cells and/or NK-cells. A method of the invention is preferably used for enhancing an antigen specific T-cell response.

A preferred marker for measuring elevation of activation and/or differentiation comprises IFNg expression of the cells that were treated by a method of the invention.
Increasing expression of said Hobit species in a Killer-cell or precursor thereof results in enhanced and/or elevated activation and/or differentiation of said Killer-cell or the progeny of said Killer-cell and/or Killer-cell progeny of said precursor. The Killer-cell or the progeny of said precursor cell preferably a naive T-cell, a memory T-cell, an NK cell, a macrophage/monocyte, an effector T-cell, an antigen-specific T-cell, preferably specific for an antigen of a tumour or a virus infected cell, and/or a CD28-CD27-CD4 positive T-cell. The cells that have become more activated and/or more differentiated are preferably memory T-cells, NK cells, effector T-cells, antigen specific T-cells and/or CD28-CD27-CD4 positive T-cells.
In another aspect the invention provides an isolated and/or recombinant nucleic acid comprising a sequence as identified in figure 8, 9 and/or 10, or a functional part, derivative and/or analogue thereof. In a preferred embodiment said nucleic acid encodes a human Hobit-L, Hobit-M, Hobit S and/or Hobit XS or a mammalian homologue thereof. A functional part is at least a part comprising the region containing the zinc fingers of a Hobit species. A derivative of a Hobit species is a Hobit species as depicted in figure 8, 9 and/or 10, having between 1 and 3 amino acid substitutions. When these amino acid substitutions are in a zinc finger it is preferred that the amino acid substitution is done with an amino acid at the corresponding position in a zinc finger of another zinc finger protein. An analogue of a Hobit species and herein preferably a human Hobit species is a human Hobit species wherein at least one and preferably at least two and more preferably at least 4 of the zinc fingers are substituted by the corresponding zinc finger or zinc fingers of a Blimp-1 protein. Preferably of a human Blimp-1 protein. Te invention thus further provides a human Hobit species wherein at least one zinc finger is replaced by another zinc finger, preferably from another zinc finger protein. In a preferred embodiment said another zinc finger protein is Blimp-1, preferably human Blimp-1.

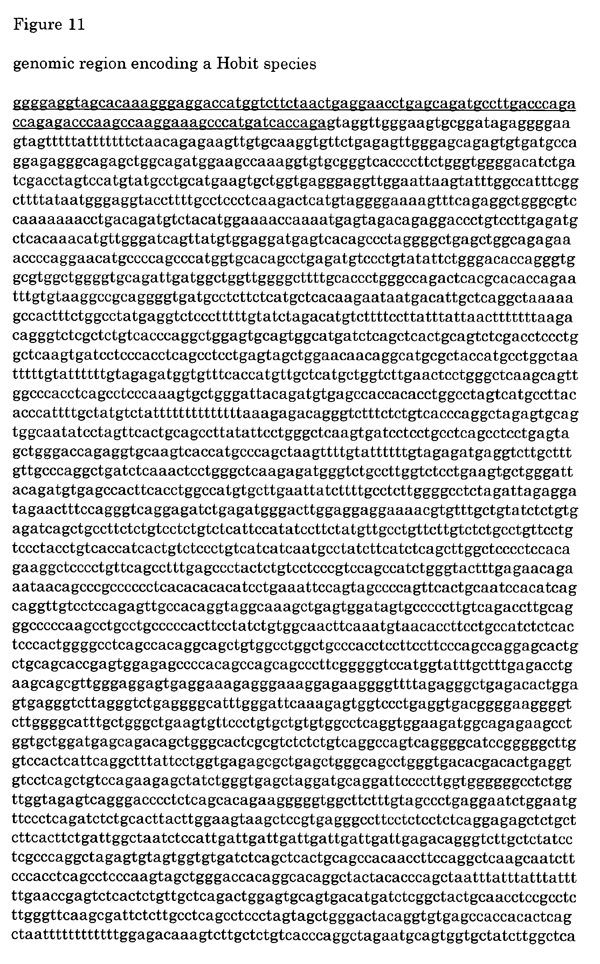

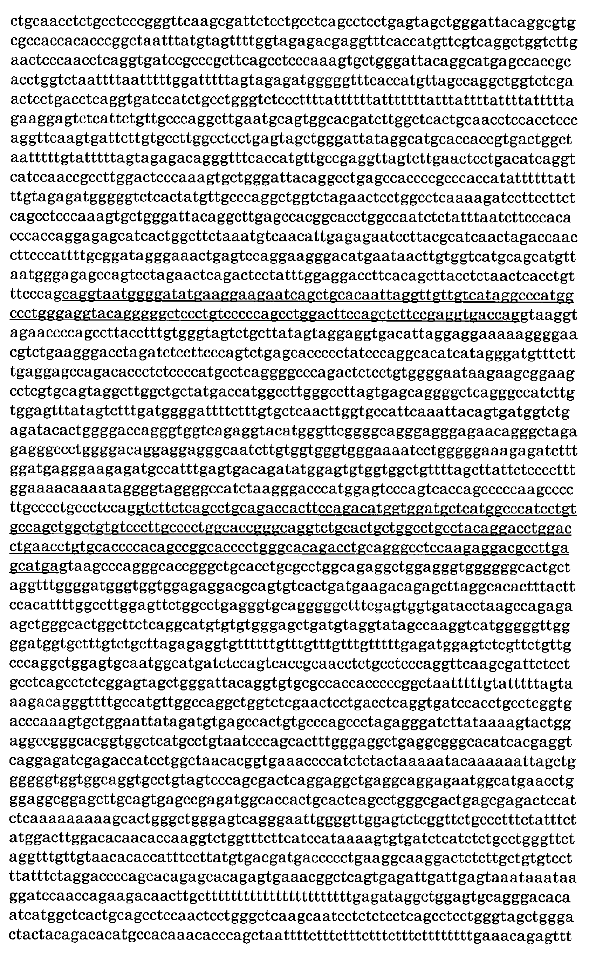

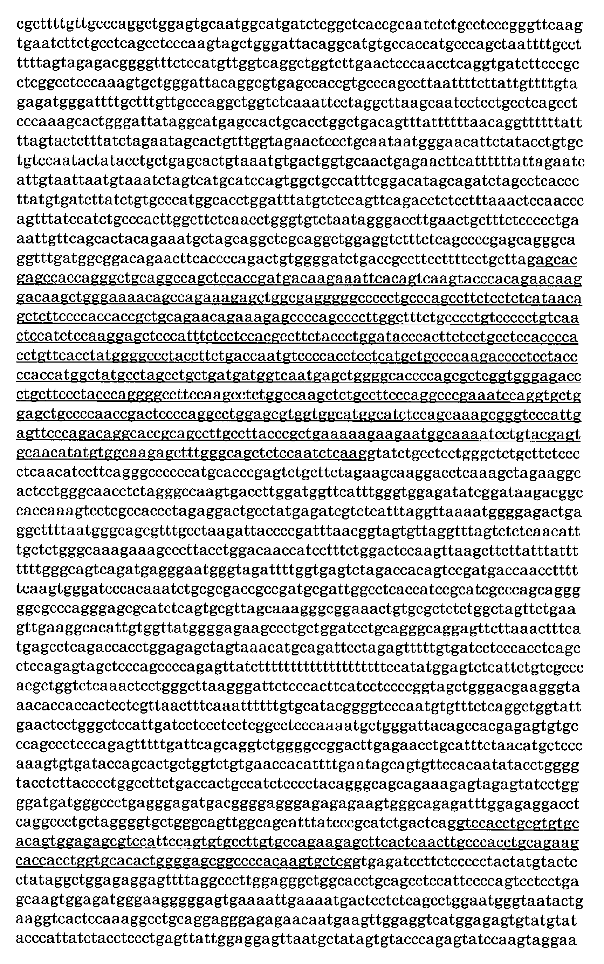

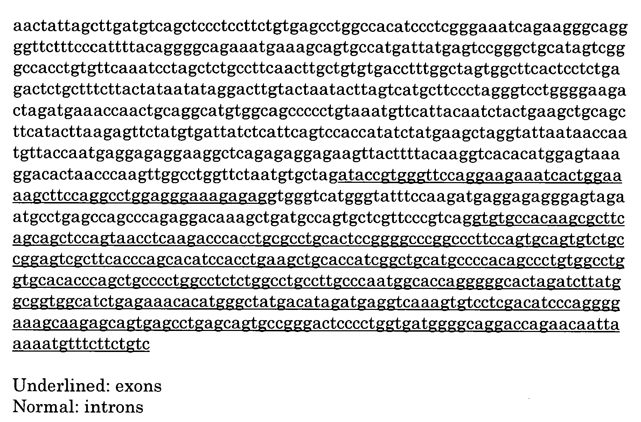

Expression of a Hobit species in a cell is preferably elevated and/or enhanced by providing said cell with a nucleic acid comprising a coding region for said Hobit species. Expression of a Hobit species in a cell that expresses said Hobit species is preferably reduced by providing said cell with a nucleic acid comprising and/or encoding an anti-sense sequence for said Hobit species. Said nucleic acid is preferably provided to said cell by means of a gene delivery vehicle comprising said nucleic acid. In one embodiment the invention therefore provides a gene delivery vehicle comprising a nucleic acid encoding a Hobit species or a functional part, derivative and/or analogue thereof. Preferably said nucleic acid encodes human Hobit-L, Hobit-M, Hobit S and/or Hobit XS or a mammalian homologue thereof. In another embodiment the invention provides a gene delivery vehicle comprising a nucleic acid or functional equivalent thereof that is or codes for an anti-sequence to a sequence as depicted in figure 8, 9 and/or 10. As mentioned herein above, there are several different types of anti-sense strategies developed at present. An antisense can be developed against the coding region, the mRNA or to the precursor RNA. The sequence of the precursor RNA is obtainable from the genomic sequence as depicted in figure 11. An antisense sequence preferably comprises a stretch of between 18 to 40 nucleotides. In a preferred embodiment said gene delivery vehicle comprises a virus particle and/or a liposome. Preferably said virus particle is a retrovirus particle, preferably a lentivirus particle, an adenovirus particle and/or an adeno-associated virus particle.

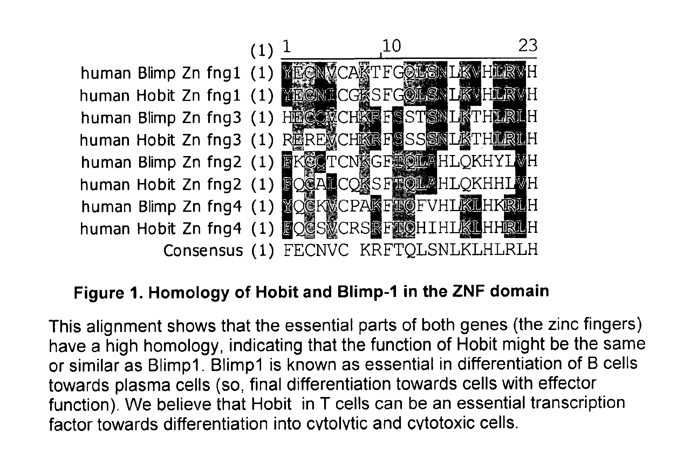

As many of the viral gene delivery vehicles require the packaged nucleic acid to at least comprise the viral packaging signal and/or a viral repeat sequence the invention further provides an isolated and/or recombinant nucleic acid of the invention, further comprising at least one virus specific sequence preferably a packaging signal and/or a virus repeat sequence. In another embodiment a nucleic acid as provided by the invention comprises at least one coding region for a virus. Preferably said packaging signal, virus repeat sequence and/or coding region is of a retrovirus, preferably a lentivirus, an adenovirus and/or an adeno-associated virus. Further provided is an isolated and/or recombinant nucleic acid according to the invention encoding a Hobit species wherein a zinc finger as identified in figure 1, is replaced by an artificial zinc finger and/or a zinc finger of a different Hobit species.

The invention further provides the use of a nucleic acid according to the invention, for altering the activation and/or differentiation state of a Killer-cell provided therewith. The invention further provides the use of a nucleic acid according to the invention, for the preparation of a vaccine. Also provided is the use of a nucleic acid according to the invention for the preparation of a medicament for altering a Killer-cell mediated immune response in an individual.

In another aspect the invention provides a method for determining the status of the Killer-cell specific immunity in an individual at the time a Killer-cell containing sample was obtained from said individual said method comprising determining in Killer-cells of said sample the expression of a Hobit species. Preferably said method further comprises comparing said expression with a reference.

### Brief description of the drawings

Figure 1
   Homology of Hobit and Blimp-1 ZNF domain.
Figure 2
   Schematic representation of Hobit and Blimp-1
Figure 3
   Hobit upregulation in the presence of IL-15
Figure 4
   Hobit upregulation in treated CD+8 cells and NK cells
Figure 5
   Increase in IFNg production in treated cells
Figure 6
   Differential gene expression in PMA/ionomycine stimulated YT2C cells after introduction of a hobit species by lentiviral transduction.
Figure 7
   Putative molecular cascade of Hobit signaling.
Figure 8
   Nucl and aa sequences of splice variants of Hobit (ZNF683).
Figure 9
   Alignment of Hobit species on nucleotide and amino acid level.
Figure 10
   Alignment of Human and mouse Hobit.
Figure 11
   Genomic region sequence of Hobit

### Examples

### Sequence of Hobit

Hobit is annotated as ZNF683. By cloning cDNA from NK cells, we found multiple splice variants of Hobit. We name the annotated form: Hobit-XL. We found several deletions: 20 nucleotides (delta 20), 60 nuceotides (delta 60), 66 nt (delta 66) and 207 nucleotides (delta 207). Up till now, we found several combinations with the following combination of deletions:

| Splicevariants | | | aminoacids | nucleotide |
|---|---|---|---|---|
| No deletions: | Extra large | Hobit-XL | 509 | 1530 |
| delta 60 alone | Large | Hobit-L | 489 | 1470 |
| delta 60 + delta 66 | Medium | Hobit-M | 467 | 1404 |
| delta 20 + delta 60 | Small | Hobit-S | 369 | 1110 |
| Delta 20 + delta 60 + delta 207 | Extra small | Hobit-XS | 300 | 903 |

In figure 8, you can find the nucleotide sequence with its translation of all splicevariants. The annotated sequence (which is similar to Hobit-XL) is named ZNF683. In figure 9, all variants are aligned on the nucleotide and aminoacid levels.

In CD8 effector-memory, CD8 memory cells, NK cells and in lung tissue we found both Hobit-XL and Hobit-L in a ratio of ~30%/70%. In testis, we found a different ratio; 50/50. In 3 large granular lymphocyte (LGL disease) samples we found ratio's of Hobit XL/L; in #1: 40/60, in #2: 30/70 in #3: 50/50. In none of the tested samples we found Hobit-S. We did not yet examine Hobit-M and Hobit-XS.

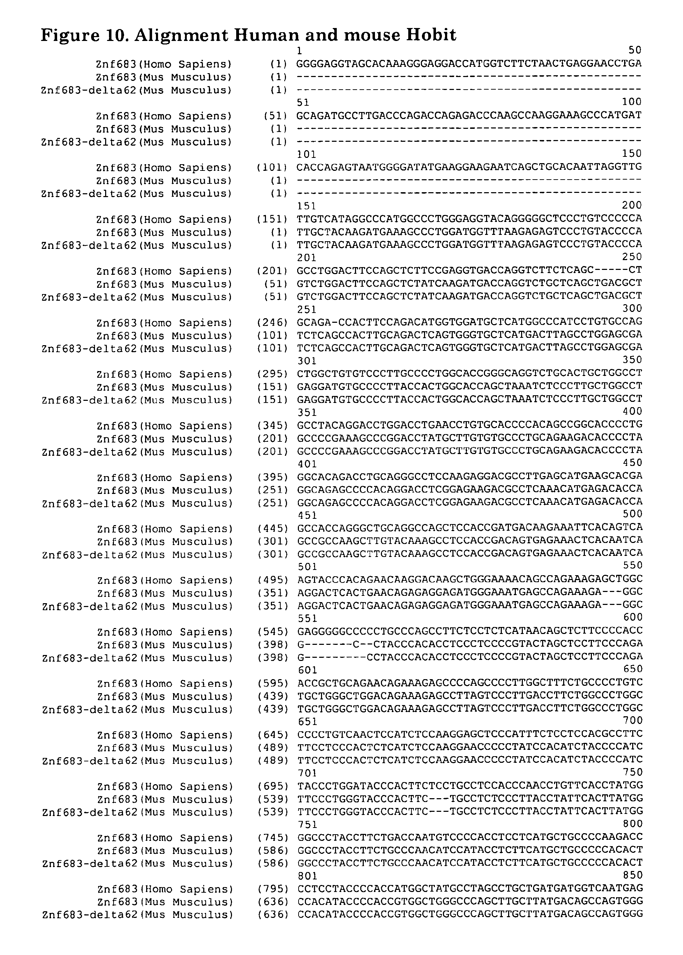

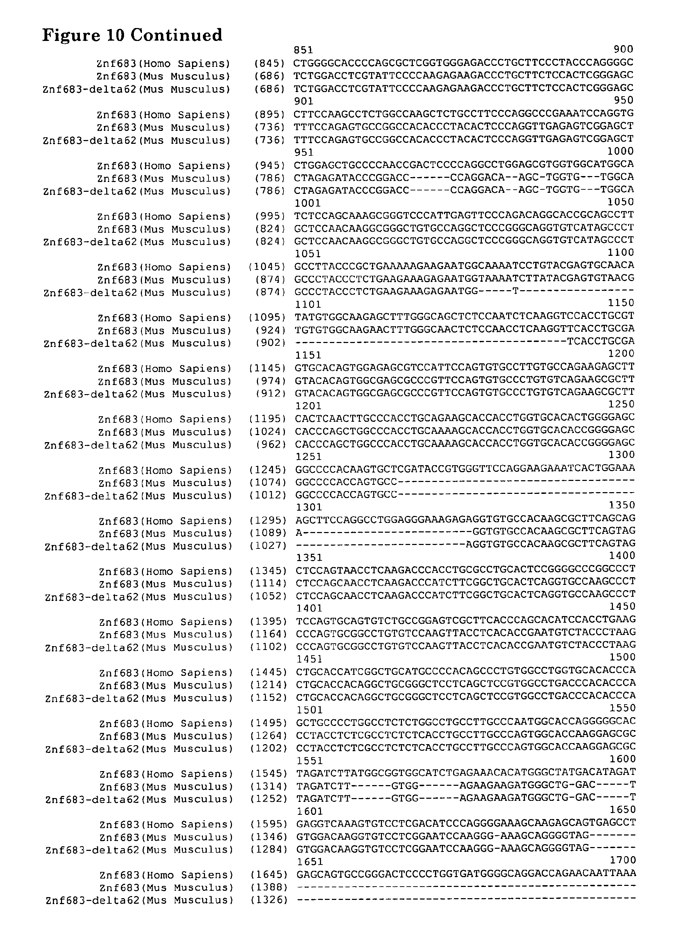

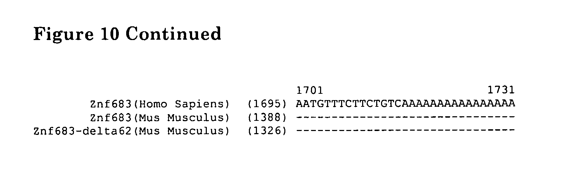

In figure 10, the alignment between human and mouse is depicted. We also found a smaller splicevariant that has a deletion of 62 nucleotides. These are approximately 65% homologous with human. The new genbank database indicates that human ZNF683 does not have a murine homologue. The murine sequences that we use to align with human ZNF683 is the sequence that we obtained from an effector-memory CD8+ T cell subpopulation (CD62L-/CD44+) from a mouse spleen.

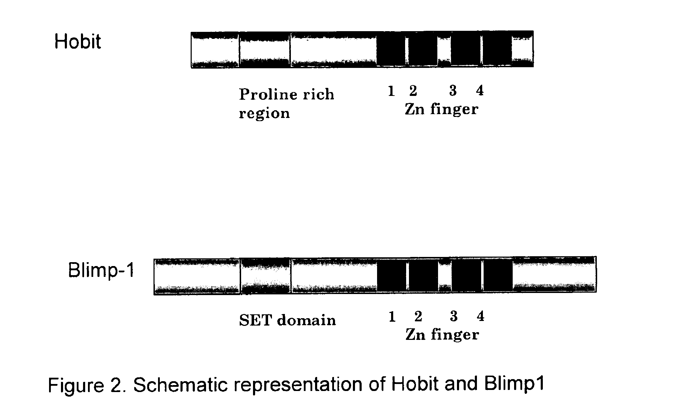

**Homology between PRDM1 (Blimp-1) and ZNF683 (Hobit) lies in the** zinc finger domain (figure 1). A simplified scheme of the essential domains present in Hobit and Blimp-1 are depicted in figure 2. In Hobit and Blimp1, the zinc finger domain consists of 4 zinc fingers of the C2H2 type. On the 5' site of the zinc finger domain, Hobit has a proline rich domain important for protein-protein interactions. In Blimp-1, there is a SET domain, also important for protein-protein interactions.

### Differential expression of Hobit

Differential expression of Hobit was found in a microarray experiment using 3 distinct CD8+ subsets (Table 1a) and in an experiment using CMV specific CD8+ T cells during a primary CMV infection (Table 1b). (M&M: microarray experiments).

### Induction of Hobit:

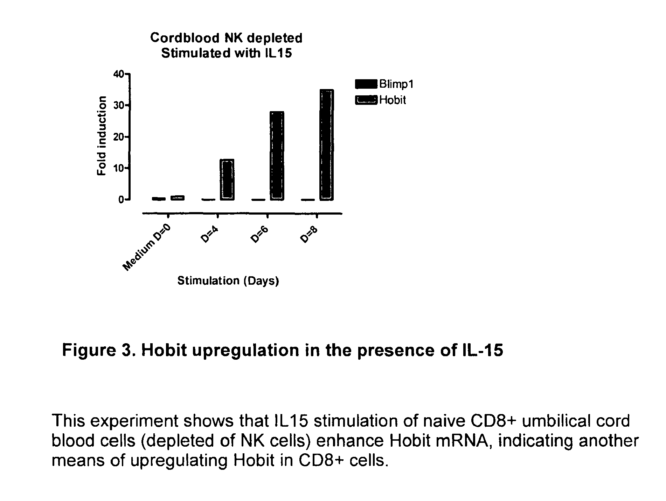

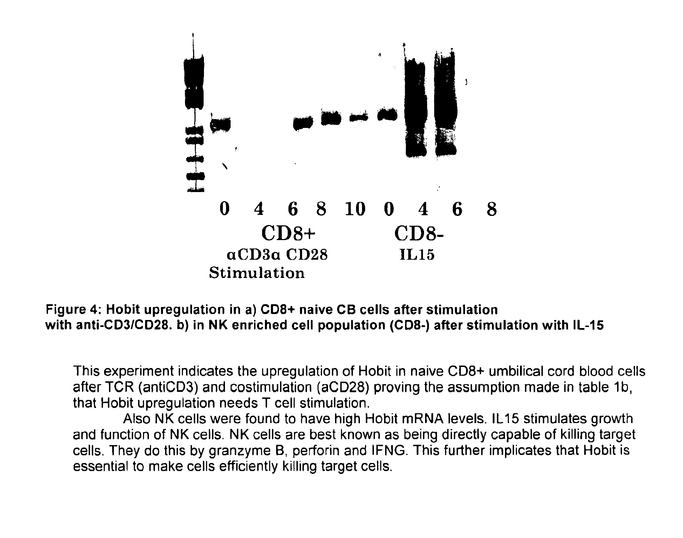

We know that Hobit can be induced
- in CD8+ naive cord blood cells cultured in the presence of IL-15. (M&M: IL-15) (figure 3)
- in CD8+ naive cord blood cells after stimulation with anti-CD3 moab in combination with anti-CD28 moab: (M&M: aCD3/aCD28) (figure 4a) (this experiment has only been performed once and need to be repeated!)
- in NK cells after stimulation with IL-15 (10 ng/ml) (Figure 4b) (M&M: Standard PCR of Hobit)

### Overexpression of Hobit (M&M: cloning of Hobit-L and M&M: Lentiviral transduction of YT2C)

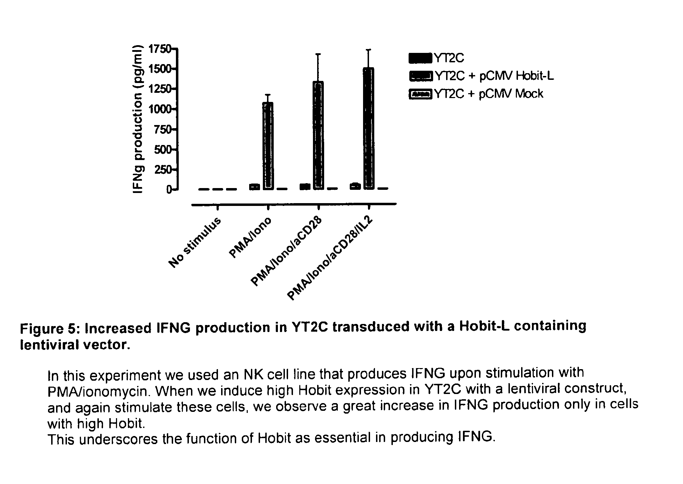

- augments IFNG production in YT2C (NK cell line) after stimulation with PMA/ionomycine in combination with anti-CD28 moab (figure 5)

### M&M: Microarray experiments

### Cell isolation of CD8+ subsets

Cells were isolated from buffy coats by a two step procedure. After Ficoll, CD8+ cells were isolated by CD8+ microbeads (Miltenyi Biotec, Utrecht, the Netherlands) and stored overnight at 4°C in serum containing medium. CD8+ cells were then stained with CD27-FITC (7C9, home-made), CD45RA-RD1 (Coulter, Miami, L) and CD8-APC (BD Pharmingen, San Diego, CA) and FACS sorted using a FACS Vantage (Beckton Dickinson (BD), San Diego, CA) in naïve CD8+ cells (CD8+CD45RA^{high}CD27^{high}), effector CD8+ cells (CD8+CD45RA^{high}CD27^{low}) and memory CD8+ cells (CD8+CD45RA^{low}CD27^{high}).

### Cell isolation of CD8+ effector cells during primary CMV infection

PBMCs from renal transplant patients were thawed and either FACS sorted (peak CMV) or isolated using the MACS beads isolation procedure (Miltenyi Biotec, Utrecht, the Netherlands) using anti-APC beads. Cells isolated 2 weeks after CMV peak were stained with HLA-DR-FITC (BD Biosciences), CD38-PE (BD Biosciences) and CD8-APC (BD Pharmingen) and HLA-DR^{high}CD38^{high}CD8⁺ were sorted using FACS Vantage (Beckton Dickinson, San Diego, CA). CMV specific cells from one year after transplantation (long term) were stained with APC conjugated tetramers loaded with pp65. After staining, tetramer+ cells were isolated by APC microbeads (Miltenyi Biotec, Utrecht, the Netherlands).

**RNA isolation Total RNA was isolated from cells with the nucleospin** RNA isolation kit (Machery-Nagel, Duren, Germany) according to manufacture's instructions. RNA Amplification, Labelling and Hybridization was performed at Service XS (Leiden, The Netherlands) with Whole human genome two-color arrays from Agilent.

### M&M Isolation and purification of cells

Human peripheral blood mononuclear cells (PBMCs) from buffy coats of healthy donors were isolated by Ficoll-Isopaque density gradient centrifugation (Nycomed; Pharma, Oslo, Norway). Umbilical cord blood mononuclear cells (UCBMCs) were used to obtain naive T cells. The study was approved by the local medical ethics committee of the Academic Medical Center.

CD8+ T cells and CD16+ NK cells were purified either from total PBMCs or UCBMCs by selection using the Dynal bead selection system and the MACS system. Brie Ly, PBMCs were stained with moabs against CD16 for 30 minutes and washed with PBA. Goat anti mouse immunoglobulines dynal beads were then incubated for 30 minutes. The Dynal magnet was applied according to the manufacturer's instruction. The CD16+ NK cells were then stored on ice for further use. The NK depleted cell population was then stained with CD8 microbeads for 15 minutes at 8°C. After washing, cells were resuspended in incubation buffer and enrichment was performed with the VarioMACS magnet according to manufacturer's instructions. The sample purity was assessed by Luorescence-activated cell sorter (FACS) with APC-conjugated CD8 and PerCP-conjugated CD3 mAbs (purity, >95% CD3+ CD8+).

### M&M IL-15

UCBMCs, or purified CD8+ T-cell subsets, were cultured for 7 days at 37°C in 5% CO2 atmosphere, in culture medium in the presence or absence of 10ng/ml IL-15 (R&D Systems).

### M&M: Standard PCR of Hobit:

Primer pairs for all variants of Hobit in a standard PCR reaction: {Hobit seq mid. Fwd}: CACCGCTGCAGAACAGAAAG & {Hobit2.0 CLN Revs} GCAAGAGCAGTGAGCTG. This amplifies a product of approximately 1100 nt.

### M&M intracellular detection of IFNG

The cytokine-producing capacity was assessed after phorbol myristate acetate (PMA; 2 ng/mL) and ionomycin (1 µM/mL) stimulation in the presence of brefeldin A (1 µM; all from Sigma Chemical, St Louis, MO) for 4 hours. Cells were fixed in 2% paraformaldehyde, permeabilized with PBS containing 0.5% (wt/vol) bovine serum albumin (BSA), and 0.5% saponin followed by staining with PE-labeled anti-IFN-y and a control mAb (gamma-1).

### Cell Culture of YT2C

YT2C is a subline of the YT cell line, originally established from a child with acute lymphoblastic lymphoma and thymoma [1]. Cell lines were cultured in Iscove's modified Dulbecco's medium (IMDM; Invitrogen), supplemented with 10% (v/v) heat-inactivated FCS (ICN Biomedicals GmbH, Meckenheim, Germany), 100 U/ml penicillin and 100 µg/ml streptomycin. 293T cells were cultured in Dulbecco's modified Eagle's medium (DMEM) containing 10% (v/v) heat-inactivated FCS (ICN Biomedicals GmbH, Meckenheim, Germany), 100 U/ml penicillin and 100 µg/ml streptomycin.

### Cloning of Hobit-L

To obtain the Hobit-L gene, the 1467 bp fragment was amplified by PCR with the following primers: GGTACCTCGCCCTGGAGGTACAGGG (Hobit4.0 CLN Forw.) and GCAAGAGCAGTGAGCTG (Hobit2.0 CLN Revs). The gene without the start codon was sequenced and cloned as a Xho1/EcoR1 fragment into pMT2SM-HA which added a HA tag in front of the open reading frame.

### Lentiviral Vector Construction and Infection

To generate the Hobit Large lentiviral construct, restriction enzymes PstI and EcoR1 were used to subclone the HA-tagged Hobit sequence into the lentiviral vector backbone RRL-cPPT-CMV-X2-PRE-SIN-IRES-eGFP (CMV). The lentiviral vector pRRLcmvgfpsin contains a phosphoglycerate kinase or cytomegalovirus promoter driving green Luorescent protein (GFP) expression. The HA-tagged Hobit Full length gene was cloned before the GFP open reading frame which yielded the new vector pRRLcmvgfpsinHobit-L (Hobit-L).

The vector pCMVHobit-L and the backbone without Hobit construct (pCMV Mock) were used in the third generation self inactivating lentiviral vector system (2, 3). Brie Ly, expression vectors for HIV Gag/Pol, HIV Rev, VSVg and the viral transfer vector were cotransfected overnight using calcium phosphate into 293T cells. The medium was changed and virus was collected twice for 24 h, passed through 0.45 µm filters and stored at -80°C.

Cells were seeded in 24 well plates at a density of 106 cells/well and incubated with virus for 4 h in the presence of 8 µg/ml polybrene and spinfection for 2 hours at 2400 rpm for 2 hours. The cells were harvested 3 days after transduction and GFP-positive cells were sorted using a FACS-Aria (BD Biosciences) cell sorter. By culturing YT2C cells tranfected with either pCMVHobit-L or pCMVMock at 1 cell per well the clone was selected with the highest GFP expression measured by Luorescent-activated cell sorting (FACS).

To assess whether overexpression of the Hobit gene had occurred, cell line YT2C was transfected with Lentivirus as described before. RNA was isolated (Machery-Nagel NucleoSpin RNA II kit) and reverse transcribed to obtain cDNA. Hobit qPCR was performed using primers CATATGTGGCAAGAGCTTTGG (Hobit RT 9.0 Forw.) and GGCAAGTTGAGTGAAGCTCT (Hobit RT 9.0 Revs.).

### In vitro Stimulation

The cytokine-producing capacity was assessed after phorbol myristate acetate (PMA; 2 ng/mL) and ionomycin (1 µM/mL)) stimulation (1 µM; all from Sigma Chemical, St Louis, MO) in the presence or absence of aCD28 (3 ug/ml) and IL2 (50 Units/ml). After 4 hours supernatants were collected and the IFNγ production was measured by for IFNγ enzyme-linked immunosorbent assay (ELISA; Sanquin, Amsterdam, the Netherlands).

### Microarray

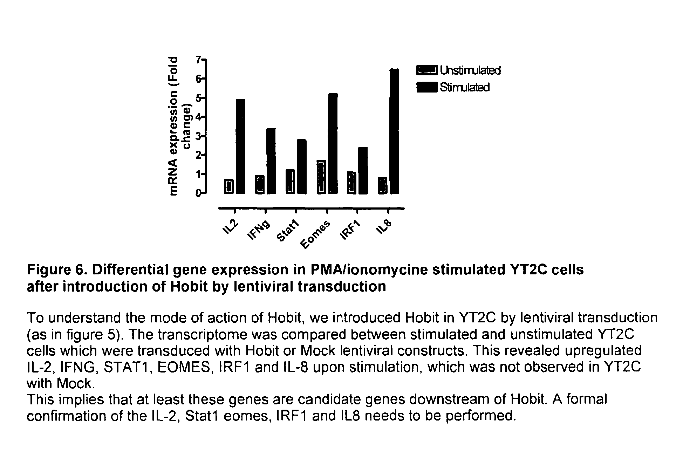

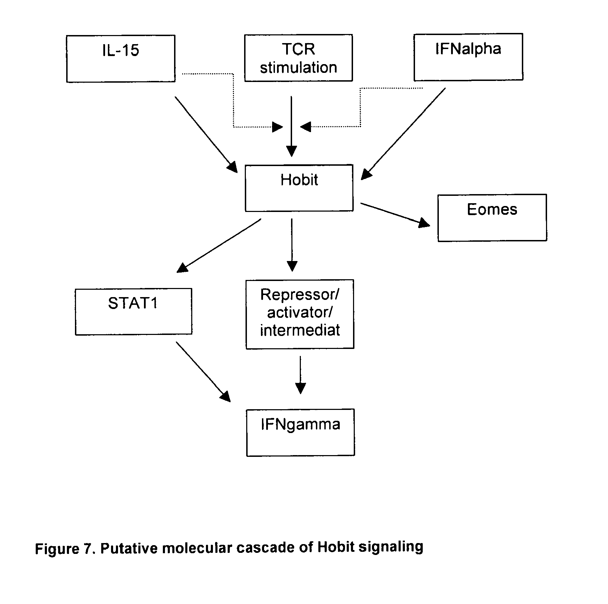

YT2C cells transfected with either pCMVHobitL or pCMV Mock were stimulated with or without PMA, Ionomycin, aCD28 as described earlier. RNA was extracted as before. Protocol micro-array Illumina microarray expression results are given in arbitrary units from Hobit-L transfected samples were divided by the numbers generated by the mock transfected YT2C cell line. The ratio between these samples is plotted in figure 6.

### References

1. Yodoi J, Teshigawara K, Nikaido T et al. TCGF (IL 2)-receptor inducing factor(s). I. Regulation of IL 2 receptor on a natural killer-like cell line (YT cells). J Immunol 1985; 134: 1623-30.
2. R. Zufferey, T. Dull, R.J. Mandel, A. Bukovsky, D. Quiroz, L. Naldini et al., Self-inactivating lentivirus vector for safe and efficient in vivo gene delivery. J Virol 1998; 72: 9873-9880.
3. T. Dull, R. Zufferey, M. Kelly, R.J. Mandel, M. Nguyen, D. Trono et al., A third-generation lentivirus vector with a conditional packaging system. J Virol 1998; 72: 8463-8471.

**Table 1a. Hobit mRNA in CD8+ subsets**

| | |
|---|---|
| Hobit in CD8+ subsets | Hobit expression (no distinction between splicevariants) |
| Subset of cells | **Fold change** Expression value of indicated subset of cellsdivided by the expression value of naive CD8+ cells |
| Naïve CD8+ cells (CD8+CD45RA+CD27+) | 1.0 |
| Effector-memory CD8+ (CD45RA+CD27-CD8+) | 17.75 |
| Memory-type CD8+ (CD45RA-CD27+CD8+) | 4.66 |

Effector-memory CD8+ cells have a high cytolytic capacity. This is evident by a high granzyme B, Perforin and IFNG content. These cells can react very quickly. These traits are opposed to the memory-type CD8+ cells that need several days to get activated (high granzymeB, Perforin and IFNG). The high Hobit mRNA expression in the CD8+ effector-memory cells indicates that Hobit is involved in the phenotype of these effector memory CD8+ cells.

**Table 1b. Hobit mRNA in primary CMV infection**

| | |
|---|---|
| Hobit in primary CMV infection | Hobit (no distinction: between splicevariants) |
| Subset of cells | **Fold change** Expression value of indicated subset of cellsdivided by the expression value of naive CD8+ cells |
| Naïve CD8+ cells (CD8+CD45RA+CD27+) | 1.10 |
| Peak of viral infection (CD8+HLA-DR+CD38+) | 11.94 |
| 1year after infection (CMV specific cells: Tetramer pp65+ CD8+ | 10.10 |
| Latency: ~ 20 years after infection:Tetramer pp65+ CD8+ | 12.06 |

This experiment shows that during an acute primary infection of CMV (Cytomegalovirus), CD8+ cells specifically reacting against CMV have high Hobit mRNA. We know that the reacting T cells are activated via TCR (CD3) and costimulation (CD28), indicating that signaling via TCR is at least one means of Hobit upregulation.

## Claims

1. A method for altering activation and/or differentiation of Killer cells comprising altering the expression of a Hobit species in said Killer-cells.

2. A method according to claim 1, wherein said activation and/or differentiation is elevated through increasing expression of said Hobit species in said Killer-cells.

3. A method according to claim 1, wherein said activation and/or differentiation is reduced through decreasing expression of said Hobit species in said Killer-cell.

4. A method according to any one of claims 1-3, wherein a Killer-cell or a Killer-cell precursor of said Killer-cell is provided with a nucleic acid for altering the expression said Hobit species in said Killer-cell or a progeny Killer-cell of said precursor.

5. A method according to claim 4, wherein said nucleic acid is provided to a naive T-cell.

6. A method according to any one of claims 1-5, wherein said Killer-cell or precursor thereof is a human Killer-cell or a human precursor thereof.

7. A method according to any one of claims 2, 4-7 wherein said expression is increased in cells in vitro.

8. A method according to claim 7, wherein said cells are a graft for transplantation comprising said Killer-cells or precursors thereof.

9. A method according to any one of claims 1-8, for enhancing a Killer-cell response of said Killer-cells or progeny thereof.

10. A method according to claim 9, for enhancing an antiviral response and/or an anti-tumour response of said Killer-cells.

11. A method according to any one of claims 1-10, for enhancing an antigen specific T-cell response, preferably an antigen-specific T-cell response specific for a tumour antigen or a virus antigen.

12. A method according to any one of claims 2, 4-11, wherein said elevating activation and/or differentiation comprises increasing interferon-gamma expression of said T-cells, increasing granzymeB or perforin expression in NK cells or another activation/differentiation marker for a Killer cell.

13. A method according to any one of claims 1-12, wherein said Killer-cells are memory T-cells, NK cells, effector T-cells, macrophages and/or CD28-CD27-CD4 positive T-cells.

14. An isolated and/or recombinant nucleic acid comprising a sequence as identified in figure 8, 9 and/or 10, or a functional part, derivative and/or analogue thereof.

15. An isolated and/or recombinant nucleic acid according to claim 14, wherein said nucleic acid encodes a human Hobit-L, Hobit-M, Hobit S and/or Hobit XS or a mammalian homologue thereof.

16. A gene delivery vehicle comprising a nucleic acid encoding a Hobit species or a functional part, derivative and/or analogue thereof.

17. A gene delivery vehicle according to claim 16, wherein said nucleic acid encodes human Hobit-L, Hobit-M, Hobit S and/or Hobit XS or a mammalian homologue thereof.

18. A gene delivery vehicle comprising a nucleic acid or functional equivalent thereof that is or codes for an anti-sequence to a sequence as depicted in figure 8, 9 and/or 10.

19. A gene delivery vehicle according to claim 17, wherein said anti-sense sequence comprises a stretch of between 18 to 40 nucleotides.

20. A gene delivery vehicle according to any one of claims 16-19, wherein said gene delivery vehicle comprises a virus particle and/or a liposome.

21. A gene delivery vehicle according to claim 20, wherein said virus particle is a retrovirus particle, preferably a lentivirus particle, an adenovirus particle and/or an adeno-associated virus particle.

22. An isolated and/or recombinant nucleic acid according to claim 14 or claim 15, further comprising at least one coding region for a virus, preferably a virus as mentioned in claim 21.

23. An isolated and/or recombinant nucleic acid according to claim 14, 15 or 22, encoding a Hobit species wherein a zinc finger of said Hobit species as identified in figure 1, is replaced by an artificial zinc finger and/or a zinc finger of a different Hobit species.

24. Use of a nucleic acid according to any one of claims 14, 15, 22 or 23, for altering the activation and/or differentiation state of a Killer-cell, preferably a T-cell, NK-cell or a macrophage, provided therewith.

25. Use of a nucleic acid according to any one of claims 14, 15, 22 or 23, for the preparation of a vaccine.

26. Use of a nucleic acid according to any one of claims 14, 15, 22 or 23, for the preparation of a medicament for altering a Killer-cell mediated immune response in an individual.

27. A method for determining the status of the Killer-cell specific immunity in an individual at the time a Killer-cell containing sample was obtained from said individual said method comprising determining in Killer-cells of said sample the expression of a Hobit species.

28. A method according to claim 27, further comprising comparing said expression with a reference.


SEQUENCE LISTING

<110> Academisch Medisch Centrum bij de Universiteit van
      Amsterdam

<120> Transcription factor for Killer cell activation, differentiation
      and uses thereof

<130> P79705EP00

<140> EP 06077192.0
<141> 2006-12-07

<160> 37

<170> PatentIn version 3.3

<210> 1
<211> 20
<212> DNA
<213> Artificial Sequence

<220>
<223> Primer M&M Standard PCR of Hobit

<400> 1
caccgctgca gaacagaaag                                                  20


<210> 2
<211> 17
<212> DNA
<213> Artificial Sequence

<220>
<223> M&M Primer Standard PCR of Hobit 2.0

<400> 2
gcaagagcag tgagctg                                                     17


<210> 3
<211> 25
<212> DNA
<213> Artificial Sequence

<220>
<223> Primer Hobil-L 4.0 CLN

<400> 3
ggtacctcgc cctggaggta caggg                                            25


<210> 4
<211> 17
<212> DNA
<213> Artificial Sequence

<220>
<223> Primer Hobit-L 2.0 CLN

<400>  4
gcaagagcag tgagctg                                                    17


<210>  5
<211>  21
<212>  DNA
<213>  Artificial Sequence

<220>
<223>  Primer Lentiviral Vector Construction and Infection Hobit RT 9.0

<400>  5
catatgtggc aagagctttg g                                               21


<210>  6
<211>  20
<212>  DNA
<213>  Artificial Sequence

<220>
<223>  Lentiviral Vector Construction and Infection Primer Hobit RT 9.0

<400>  6
ggcaagttga gtgaagctct                                                 20


<210>  7
<211>  23
<212>  PRT
<213>  Homo sapiens

<400>  7

Tyr Glu Cys Asn Val Cys Ala Lys Thr Phe Gly Gln Leu Ser Asn Leu
1               5                   10                  15


Lys Val His Leu Arg Val His
            20


<210>  8
<211>  23
<212>  PRT
<213>  Homo sapiens

<400>  8

Tyr Glu Cys Asn Ile Cys Gly Lys Ser Phe Gly Gln Leu Ser Asn Leu
1               5                   10                  15


Lys Val His Leu Arg Val His
            20


<210>  9
<211>  23
<212>  PRT

<213>  Homo sapiens

<400>  9

His Glu Cys Gln Val Cys His Lys Arg Phe Ser Ser Thr Ser Asn Leu
1               5                   10                  15

Lys Thr His Leu Arg Leu His
            20

<210>  10
<211>  23
<212>  PRT
<213>  Homo sapiens

<400>  10

Arg Glu Arg Glu Val Cys His Lys Arg Phe Ser Ser Ser Ser Asn Leu
1               5                   10                  15

Lys Thr His Leu Arg Leu His
            20

<210>  11
<211>  23
<212>  PRT
<213>  Homo sapiens

<400>  11

Phe Lys Cys Gln Thr Cys Asn Lys Gly Phe Thr Gln Leu Ala His Leu
1               5                   10                  15

Gln Lys His Tyr Leu Val His
            20

<210>  12
<211>  23
<212>  PRT
<213>  Homo sapiens

<400>  12

Phe Gln Cys Ala Leu Cys Gln Lys Ser Phe Thr Gln Leu Ala His Leu
1               5                   10                  15

Gln Lys His His Leu Val His
            20

<210>  13
<211>  23
<212>  PRT
<213>  Homo sapiens

<400>  13

Tyr Gln Cys Lys Val Cys Pro Ala Lys Phe Thr Gln Phe Val His Leu
1               5                   10                  15

Lys Leu His Lys Arg Leu His
            20

<210>  14
<211>  23
<212>  PRT
<213>  Homo sapiens

<400>  14

Phe Gln Cys Ser Val Cys Arg Ser Arg Phe Thr Gln His Ile His Leu
1               5                   10                  15

Lys Leu His His Arg Leu His
            20

<210>  15
<211>  23
<212>  PRT
<213>  Artificial sequence

<220>
<223>  Consensus sequence zinc finger Hobit

<220>
<221>  VARIANT
<222>  (7)..(7)
<223>  Ala, Gly, Asn, Gln, Pro or Arg

<400>  15

Phe Glu Cys Asn Val Cys His Lys Arg Phe Thr Gln Leu Ser Asn Leu
1               5                   10                  15

Lys Leu His Leu Arg Leu His
            20

<210>  16
<211>  1530
<212>  DNA
<213>  Homo sapiens

<220>
<221>  CDS
<222>  (1)..(1530)

<400>  16

atg gcc ctg gga ggt aca ggg ggc tcc ctg tcc ccc agc ctg gac ttc 48
Met Ala Leu Gly Gly Thr Gly Gly Ser Leu Ser Pro Ser Leu Asp Phe
1               5                   10                  15

cag ctc ttc cga ggt gac cag gtc ttc tca gcc tgc aga cca ctt cca 96
Gln Leu Phe Arg Gly Asp Gln Val Phe Ser Ala Cys Arg Pro Leu Pro
            20                  25                  30

gac atg gtg gat gct cat ggc cca tcc tgt gcc agc tgg ctg tgt ccc 144
Asp Met Val Asp Ala His Gly Pro Ser Cys Ala Ser Trp Leu Cys Pro
        35                  40                  45

ttg ccc ctg gca ccg ggc agg tct gca ctg ctg gcc tgc cta cag gac 192
Leu Pro Leu Ala Pro Gly Arg Ser Ala Leu Leu Ala Cys Leu Gln Asp
    50                  55                  60

ctg gac ctg aac ctg tgc acc cca cag ccg gca ccc ctg ggc aca gac 240
Leu Asp Leu Asn Leu Cys Thr Pro Gln Pro Ala Pro Leu Gly Thr Asp
65                  70                  75                  80

ctg cag ggc ctc caa gag gac gcc ttg agc atg aag cac gag cca cca 288
Leu Gln Gly Leu Gln Glu Asp Ala Leu Ser Met Lys His Glu Pro Pro
                85                  90                  95

ggg ctg cag gcc agc tcc acc gat gac aag aaa ttc aca gtc aag tac 336
Gly Leu Gln Ala Ser Ser Thr Asp Asp Lys Lys Phe Thr Val Lys Tyr
            100                 105                 110

cca cag aac aag gac aag ctg gga aaa cag cca gaa aga gct ggc gag 384
Pro Gln Asn Lys Asp Lys Leu Gly Lys Gln Pro Glu Arg Ala Gly Glu
        115                 120                 125

ggg gcc ccc tgc cca gcc ttc tcc tct cat aac agc tct tcc cca cca 432
Gly Ala Pro Cys Pro Ala Phe Ser Ser His Asn Ser Ser Ser Pro Pro
    130                 135                 140

ccg ctg cag aac aga aag agc ccc agc ccc ttg gct ttc tgc ccc tgt 480
Pro Leu Gln Asn Arg Lys Ser Pro Ser Pro Leu Ala Phe Cys Pro Cys
145                 150                 155                 160

ccc cct gtc aac tcc atc tcc aag gag ctc cca ttt ctc ctc cac gcc 528
Pro Pro Val Asn Ser Ile Ser Lys Glu Leu Pro Phe Leu Leu His Ala
                165                 170                 175

ttc tac cct gga tac cca ctt ctc ctg cct cca ccc aac ctg ttc acc 576
Phe Tyr Pro Gly Tyr Pro Leu Leu Leu Pro Pro Pro Asn Leu Phe Thr
            180                 185                 190

tat ggg gcc cta cct tct gac caa tgt ccc cac ctc ctc atg ctg ccc 624
Tyr Gly Ala Leu Pro Ser Asp Gln Cys Pro His Leu Leu Met Leu Pro
        195                 200                 205

caa gac ccc tcc tac ccc acc atg gct atg cct agc ctg ctg atg atg 672
Gln Asp Pro Ser Tyr Pro Thr Met Ala Met Pro Ser Leu Leu Met Met
    210                 215                 220

gtc aat gag ctg ggg cac ccc agc gct cgg tgg gag acc ctg ctt ccc 720
Val Asn Glu Leu Gly His Pro Ser Ala Arg Trp Glu Thr Leu Leu Pro
225                 230                 235                 240

tac cca ggg gcc ttc caa gcc tct ggc caa gct ctg cct tcc cag gcc 768

Tyr Pro Gly Ala Phe Gln Ala Ser Gly Gln Ala Leu Pro Ser Gln Ala
                245                 250                 255

cga aat cca ggt gct gga gct gcc cca acc gac tcc cca ggc ctg gag      816
Arg Asn Pro Gly Ala Gly Ala Ala Pro Thr Asp Ser Pro Gly Leu Glu
            260                 265                 270

cgt ggt ggc atg gca tct cca gca aag cgg gtc cca ttg agt tcc cag      864
Arg Gly Gly Met Ala Ser Pro Ala Lys Arg Val Pro Leu Ser Ser Gln
        275                 280                 285

aca ggc acc gca gcc ttg cct tac ccg ctg aaa aag aag aat ggc aaa      912
Thr Gly Thr Ala Ala Leu Pro Tyr Pro Leu Lys Lys Lys Asn Gly Lys
    290                 295                 300

atc ctg tac gag tgc aac ata tgt ggc aag agc ttt ggg cag ctc tcc      960
Ile Leu Tyr Glu Cys Asn Ile Cys Gly Lys Ser Phe Gly Gln Leu Ser
305                 310                 315                 320

aat ctc aag gtc cac ctg cgt gtg cac agt gga gag cgt cca ttc cag     1008
Asn Leu Lys Val His Leu Arg Val His Ser Gly Glu Arg Pro Phe Gln
                325                 330                 335

tgt gcc ttg tgc cag aag agc ttc act caa ctt gcc cac ctg cag aag     1056
Cys Ala Leu Cys Gln Lys Ser Phe Thr Gln Leu Ala His Leu Gln Lys
            340                 345                 350

cac cac ctg gtg cac act ggg gag cgg ccc cac aag tgc tcg ata ccg     1104
His His Leu Val His Thr Gly Glu Arg Pro His Lys Cys Ser Ile Pro
        355                 360                 365

tgg gtt cca gga aga aat cac tgg aaa agc ttc cag gcc tgg agg gaa     1152
Trp Val Pro Gly Arg Asn His Trp Lys Ser Phe Gln Ala Trp Arg Glu
    370                 375                 380

aga gag gtg tgc cac aag cgc ttc agc agc tcc agt aac ctc aag acc     1200
Arg Glu Val Cys His Lys Arg Phe Ser Ser Ser Ser Asn Leu Lys Thr
385                 390                 395                 400

cac ctg cgc ctg cac tcc ggg gcc cgg ccc ttc cag tgc agt gtc tgc     1248
His Leu Arg Leu His Ser Gly Ala Arg Pro Phe Gln Cys Ser Val Cys
                405                 410                 415

cgg agt cgc ttc acc cag cac atc cac ctg aag ctg cac cat cgg ctg     1296
Arg Ser Arg Phe Thr Gln His Ile His Leu Lys Leu His His Arg Leu
            420                 425                 430

cat gcc cca cag ccc tgt ggc ctg gtg cac acc cag ctg ccc ctg gcc     1344
His Ala Pro Gln Pro Cys Gly Leu Val His Thr Gln Leu Pro Leu Ala
        435                 440                 445

tct ctg gcc tgc ctt gcc caa tgg cac cag ggg gca cta gat ctt atg     1392
Ser Leu Ala Cys Leu Ala Gln Trp His Gln Gly Ala Leu Asp Leu Met
    450                 455                 460

gcg gtg gca tct gag aaa cac atg ggc tat gac ata gat gag gtc aaa     1440
Ala Val Ala Ser Glu Lys His Met Gly Tyr Asp Ile Asp Glu Val Lys
465                 470                 475                 480

gtg tcc tcg aca tcc cag ggg aaa gca aga gca gtg agc ctg agc agt     1488
Val Ser Ser Thr Ser Gln Gly Lys Ala Arg Ala Val Ser Leu Ser Ser

485                 490                 495

gcc ggg act ccc ctg gtg atg ggg cag gac cag aac aat taa                1530
Ala Gly Thr Pro Leu Val Met Gly Gln Asp Gln Asn Asn
            500                 505

<210>  17
<211>  509
<212>  PRT
<213>  Homo sapiens

<400>  17

Met Ala Leu Gly Gly Thr Gly Gly Ser Leu Ser Pro Ser Leu Asp Phe
1               5                   10                  15

Gln Leu Phe Arg Gly Asp Gln Val Phe Ser Ala Cys Arg Pro Leu Pro
            20                  25                  30

Asp Met Val Asp Ala His Gly Pro Ser Cys Ala Ser Trp Leu Cys Pro
        35                  40                  45

Leu Pro Leu Ala Pro Gly Arg Ser Ala Leu Leu Ala Cys Leu Gln Asp
    50                  55                  60

Leu Asp Leu Asn Leu Cys Thr Pro Gln Pro Ala Pro Leu Gly Thr Asp
65                  70                  75                  80

Leu Gln Gly Leu Gln Glu Asp Ala Leu Ser Met Lys His Glu Pro Pro
                85                  90                  95

Gly Leu Gln Ala Ser Ser Thr Asp Asp Lys Lys Phe Thr Val Lys Tyr
            100                 105                 110

Pro Gln Asn Lys Asp Lys Leu Gly Lys Gln Pro Glu Arg Ala Gly Glu
        115                 120                 125

Gly Ala Pro Cys Pro Ala Phe Ser Ser His Asn Ser Ser Ser Pro Pro
    130                 135                 140

Pro Leu Gln Asn Arg Lys Ser Pro Ser Pro Leu Ala Phe Cys Pro Cys
145                 150                 155                 160

Pro Pro Val Asn Ser Ile Ser Lys Glu Leu Pro Phe Leu Leu His Ala
                165                 170                 175

Phe Tyr Pro Gly Tyr Pro Leu Leu Leu Pro Pro Pro Asn Leu Phe Thr
            180                 185                 190

Tyr Gly Ala Leu Pro Ser Asp Gln Cys Pro His Leu Leu Met Leu Pro
        195                 200                 205

Gln Asp Pro Ser Tyr Pro Thr Met Ala Met Pro Ser Leu Leu Met Met
    210                 215                 220

Val Asn Glu Leu Gly His Pro Ser Ala Arg Trp Glu Thr Leu Leu Pro
225                 230                 235                 240

Tyr Pro Gly Ala Phe Gln Ala Ser Gly Gln Ala Leu Pro Ser Gln Ala
                245                 250                 255

Arg Asn Pro Gly Ala Gly Ala Ala Pro Thr Asp Ser Pro Gly Leu Glu
            260                 265                 270

Arg Gly Gly Met Ala Ser Pro Ala Lys Arg Val Pro Leu Ser Ser Gln
        275                 280                 285

Thr Gly Thr Ala Ala Leu Pro Tyr Pro Leu Lys Lys Lys Asn Gly Lys
    290                 295                 300

Ile Leu Tyr Glu Cys Asn Ile Cys Gly Lys Ser Phe Gly Gln Leu Ser
305                 310                 315                 320

Asn Leu Lys Val His Leu Arg Val His Ser Gly Glu Arg Pro Phe Gln
                325                 330                 335

Cys Ala Leu Cys Gln Lys Ser Phe Thr Gln Leu Ala His Leu Gln Lys
            340                 345                 350

His His Leu Val His Thr Gly Glu Arg Pro His Lys Cys Ser Ile Pro
        355                 360                 365

Trp Val Pro Gly Arg Asn His Trp Lys Ser Phe Gln Ala Trp Arg Glu
    370                 375                 380

Arg Glu Val Cys His Lys Arg Phe Ser Ser Ser Ser Asn Leu Lys Thr
385                 390                 395                 400

His Leu Arg Leu His Ser Gly Ala Arg Pro Phe Gln Cys Ser Val Cys
                405                 410                 415

Arg Ser Arg Phe Thr Gln His Ile His Leu Lys Leu His His Arg Leu
            420                 425                 430

His Ala Pro Gln Pro Cys Gly Leu Val His Thr Gln Leu Pro Leu Ala
        435                 440                 445

Ser Leu Ala Cys Leu Ala Gln Trp His Gln Gly Ala Leu Asp Leu Met
    450                 455                 460

Ala Val Ala Ser Glu Lys His Met Gly Tyr Asp Ile Asp Glu Val Lys
465                 470                 475                 480

Val Ser Ser Thr Ser Gln Gly Lys Ala Arg Ala Val Ser Leu Ser Ser
                485                 490                 495

Ala Gly Thr Pro Leu Val Met Gly Gln Asp Gln Asn Asn
            500                 505

<210>  18
<211>  1530
<212>  DNA
<213>  Homo sapiens

<220>
<221>  CDS
<222>  (1)..(1530)

<400>  18
atg gcc ctg gga ggt aca ggg ggc tcc ctg tcc ccc agc ctg gac ttc      48
Met Ala Leu Gly Gly Thr Gly Gly Ser Leu Ser Pro Ser Leu Asp Phe
1               5                   10                  15

cag ctc ttc cga ggt gac cag gtc ttc tca gcc tgc aga cca ctt cca      96
Gln Leu Phe Arg Gly Asp Gln Val Phe Ser Ala Cys Arg Pro Leu Pro
            20                  25                  30

gac atg gtg gat gct cat ggc cca tcc tgt gcc agc tgg ctg tgt ccc      144
Asp Met Val Asp Ala His Gly Pro Ser Cys Ala Ser Trp Leu Cys Pro
        35                  40                  45

ttg ccc ctg gca ccg ggc agg tct gca ctg ctg gcc tgc cta cag gac      192
Leu Pro Leu Ala Pro Gly Arg Ser Ala Leu Leu Ala Cys Leu Gln Asp
    50                  55                  60

ctg gac ctg aac ctg tgc acc cca cag ccg gca ccc ctg ggc aca gac      240
Leu Asp Leu Asn Leu Cys Thr Pro Gln Pro Ala Pro Leu Gly Thr Asp
65                  70                  75                  80

ctg cag ggc ctc caa gag gac gcc ttg agc atg aag cac gag cca cca      288
Leu Gln Gly Leu Gln Glu Asp Ala Leu Ser Met Lys His Glu Pro Pro
                85                  90                  95

ggg ctg cag gcc agc tcc acc gat gac aag aaa ttc aca gtc aag tac      336
Gly Leu Gln Ala Ser Ser Thr Asp Asp Lys Lys Phe Thr Val Lys Tyr
            100                 105                 110

cca cag aac aag gac aag ctg gga aaa cag cca gaa aga gct ggc gag      384
Pro Gln Asn Lys Asp Lys Leu Gly Lys Gln Pro Glu Arg Ala Gly Glu

115                 120                 125

ggg gcc ccc tgc cca gcc ttc tcc tct cat aac agc tct tcc cca cca      432
Gly Ala Pro Cys Pro Ala Phe Ser Ser His Asn Ser Ser Ser Pro Pro
    130                 135                 140

ccg ctg cag aac aga aag agc ccc agc ccc ttg gct ttc tgc ccc tgt      480
Pro Leu Gln Asn Arg Lys Ser Pro Ser Pro Leu Ala Phe Cys Pro Cys
145                 150                 155                 160

ccc cct gtc aac tcc atc tcc aag gag ctc cca ttt ctc ctc cac gcc      528
Pro Pro Val Asn Ser Ile Ser Lys Glu Leu Pro Phe Leu Leu His Ala
                165                 170                 175

ttc tac cct gga tac cca ctt ctc ctg cct cca ccc aac ctg ttc acc      576
Phe Tyr Pro Gly Tyr Pro Leu Leu Leu Pro Pro Pro Asn Leu Phe Thr
            180                 185                 190

tat ggg gcc cta cct tct gac caa tgt ccc cac ctc ctc atg ctg ccc      624
Tyr Gly Ala Leu Pro Ser Asp Gln Cys Pro His Leu Leu Met Leu Pro
        195                 200                 205

caa gac ccc tcc tac ccc acc atg gct atg cct agc ctg ctg atg atg      672
Gln Asp Pro Ser Tyr Pro Thr Met Ala Met Pro Ser Leu Leu Met Met
    210                 215                 220

gtc aat gag ctg ggg cac ccc agc gct cgg tgg gag acc ctg ctt ccc      720
Val Asn Glu Leu Gly His Pro Ser Ala Arg Trp Glu Thr Leu Leu Pro
225                 230                 235                 240

tac cca ggg gcc ttc caa gcc tct ggc caa gct ctg cct tcc cag gcc      768
Tyr Pro Gly Ala Phe Gln Ala Ser Gly Gln Ala Leu Pro Ser Gln Ala
                245                 250                 255

cga aat cca ggt gct gga gct gcc cca acc gac tcc cca ggc ctg gag      816
Arg Asn Pro Gly Ala Gly Ala Ala Pro Thr Asp Ser Pro Gly Leu Glu
            260                 265                 270

cgt ggt ggc atg gca tct cca gca aag cgg gtc cca ttg agt tcc cag      864
Arg Gly Gly Met Ala Ser Pro Ala Lys Arg Val Pro Leu Ser Ser Gln
        275                 280                 285

aca ggc acc gca gcc ttg cct tac ccg ctg aaa aag aag aat ggc aaa      912
Thr Gly Thr Ala Ala Leu Pro Tyr Pro Leu Lys Lys Lys Asn Gly Lys
    290                 295                 300

atc ctg tac gag tgc aac ata tgt ggc aag agc ttt ggg cag ctc tcc      960
Ile Leu Tyr Glu Cys Asn Ile Cys Gly Lys Ser Phe Gly Gln Leu Ser
305                 310                 315                 320

aat ctc aag gtc cac ctg cgt gtg cac agt gga gag cgt cca ttc cag     1008
Asn Leu Lys Val His Leu Arg Val His Ser Gly Glu Arg Pro Phe Gln
                325                 330                 335

tgt gcc ttg tgc cag aag agc ttc act caa ctt gcc cac ctg cag aag     1056
Cys Ala Leu Cys Gln Lys Ser Phe Thr Gln Leu Ala His Leu Gln Lys
            340                 345                 350

cac cac ctg gtg cac act ggg gag cgg ccc cac aag tgc tcg ata ccg     1104
His His Leu Val His Thr Gly Glu Arg Pro His Lys Cys Ser Ile Pro
        355                 360                 365

tgg gtt cca gga aga aat cac tgg aaa agc ttc cag gcc tgg agg gaa     1152
Trp Val Pro Gly Arg Asn His Trp Lys Ser Phe Gln Ala Trp Arg Glu
    370                 375                 380

aga gag gtg tgc cac aag cgc ttc agc agc tcc agt aac ctc aag acc     1200
Arg Glu Val Cys His Lys Arg Phe Ser Ser Ser Ser Asn Leu Lys Thr
385                 390                 395                 400

cac ctg cgc ctg cac tcc ggg gcc cgg ccc ttc cag tgc agt gtc tgc     1248
His Leu Arg Leu His Ser Gly Ala Arg Pro Phe Gln Cys Ser Val Cys
                405                 410                 415

cgg agt cgc ttc acc cag cac atc cac ctg aag ctg cac cat cgg ctg     1296
Arg Ser Arg Phe Thr Gln His Ile His Leu Lys Leu His His Arg Leu
            420                 425                 430

cat gcc cca cag ccc tgt ggc ctg gtg cac acc cag ctg ccc ctg gcc     1344
His Ala Pro Gln Pro Cys Gly Leu Val His Thr Gln Leu Pro Leu Ala
        435                 440                 445

tct ctg gcc tgc ctt gcc caa tgg cac cag ggg gca cta gat ctt atg     1392
Ser Leu Ala Cys Leu Ala Gln Trp His Gln Gly Ala Leu Asp Leu Met
    450                 455                 460

gcg gtg gca tct gag aaa cac atg ggc tat gac ata gat gag gtc aaa     1440
Ala Val Ala Ser Glu Lys His Met Gly Tyr Asp Ile Asp Glu Val Lys
465                 470                 475                 480

gtg tcc tcg aca tcc cag ggg aaa gca aga gca gtg agc ctg agc agt     1488
Val Ser Ser Thr Ser Gln Gly Lys Ala Arg Ala Val Ser Leu Ser Ser
                485                 490                 495

gcc ggg act ccc ctg gtg atg ggg cag gac cag aac aat taa             1530
Ala Gly Thr Pro Leu Val Met Gly Gln Asp Gln Asn Asn
            500                 505


<210>  19
<211>  509
<212>  PRT
<213>  Homo sapiens

<400>  19

Met Ala Leu Gly Gly Thr Gly Gly Ser Leu Ser Pro Ser Leu Asp Phe
1               5                   10                  15


Gln Leu Phe Arg Gly Asp Gln Val Phe Ser Ala Cys Arg Pro Leu Pro
            20                  25                  30


Asp Met Val Asp Ala His Gly Pro Ser Cys Ala Ser Trp Leu Cys Pro
        35                  40                  45


Leu Pro Leu Ala Pro Gly Arg Ser Ala Leu Leu Ala Cys Leu Gln Asp
    50                  55                  60

Leu Asp Leu Asn Leu Cys Thr Pro Gln Pro Ala Pro Leu Gly Thr Asp
65                  70                  75                  80

Leu Gln Gly Leu Gln Glu Asp Ala Leu Ser Met Lys His Glu Pro Pro
                85                  90                  95

Gly Leu Gln Ala Ser Ser Thr Asp Asp Lys Lys Phe Thr Val Lys Tyr
            100                 105                 110

Pro Gln Asn Lys Asp Lys Leu Gly Lys Gln Pro Glu Arg Ala Gly Glu
        115                 120                 125

Gly Ala Pro Cys Pro Ala Phe Ser Ser His Asn Ser Ser Ser Pro Pro
    130                 135                 140

Pro Leu Gln Asn Arg Lys Ser Pro Ser Pro Leu Ala Phe Cys Pro Cys
145                 150                 155                 160

Pro Pro Val Asn Ser Ile Ser Lys Glu Leu Pro Phe Leu Leu His Ala
                165                 170                 175

Phe Tyr Pro Gly Tyr Pro Leu Leu Leu Pro Pro Pro Asn Leu Phe Thr
            180                 185                 190

Tyr Gly Ala Leu Pro Ser Asp Gln Cys Pro His Leu Leu Met Leu Pro
        195                 200                 205

Gln Asp Pro Ser Tyr Pro Thr Met Ala Met Pro Ser Leu Leu Met Met
    210                 215                 220

Val Asn Glu Leu Gly His Pro Ser Ala Arg Trp Glu Thr Leu Leu Pro
225                 230                 235                 240

Tyr Pro Gly Ala Phe Gln Ala Ser Gly Gln Ala Leu Pro Ser Gln Ala
                245                 250                 255

Arg Asn Pro Gly Ala Gly Ala Ala Pro Thr Asp Ser Pro Gly Leu Glu
            260                 265                 270

Arg Gly Gly Met Ala Ser Pro Ala Lys Arg Val Pro Leu Ser Ser Gln
        275                 280                 285

Thr Gly Thr Ala Ala Leu Pro Tyr Pro Leu Lys Lys Lys Asn Gly Lys
    290                 295                 300

Ile Leu Tyr Glu Cys Asn Ile Cys Gly Lys Ser Phe Gly Gln Leu Ser

305                 310                 315                 320

Asn Leu Lys Val His Leu Arg Val His Ser Gly Glu Arg Pro Phe Gln
                325                 330                 335

Cys Ala Leu Cys Gln Lys Ser Phe Thr Gln Leu Ala His Leu Gln Lys
            340                 345                 350

His His Leu Val His Thr Gly Glu Arg Pro His Lys Cys Ser Ile Pro
        355                 360                 365

Trp Val Pro Gly Arg Asn His Trp Lys Ser Phe Gln Ala Trp Arg Glu
    370                 375                 380

Arg Glu Val Cys His Lys Arg Phe Ser Ser Ser Ser Asn Leu Lys Thr
385                 390                 395                 400

His Leu Arg Leu His Ser Gly Ala Arg Pro Phe Gln Cys Ser Val Cys
                405                 410                 415

Arg Ser Arg Phe Thr Gln His Ile His Leu Lys Leu His His Arg Leu
            420                 425                 430

His Ala Pro Gln Pro Cys Gly Leu Val His Thr Gln Leu Pro Leu Ala
        435                 440                 445

Ser Leu Ala Cys Leu Ala Gln Trp His Gln Gly Ala Leu Asp Leu Met
    450                 455                 460

Ala Val Ala Ser Glu Lys His Met Gly Tyr Asp Ile Asp Glu Val Lys
465                 470                 475                 480

Val Ser Ser Thr Ser Gln Gly Lys Ala Arg Ala Val Ser Leu Ser Ser
                485                 490                 495

Ala Gly Thr Pro Leu Val Met Gly Gln Asp Gln Asn Asn
            500                 505

<210>  20
<211>  1470
<212>  DNA
<213>  Homo sapiens

<220>
<221>  CDS
<222>  (1)..(1470)

<400> 20
atg gcc ctg gga ggt aca ggg ggc tcc ctg tcc ccc agc ctg gac ttc 48
Met Ala Leu Gly Gly Thr Gly Gly Ser Leu Ser Pro Ser Leu Asp Phe
1               5                   10                  15

cag ctc ttc cga ggt gac cag gtc ttc tca gcc tgc aga cca ctt cca 96
Gln Leu Phe Arg Gly Asp Gln Val Phe Ser Ala Cys Arg Pro Leu Pro
            20                  25                  30

gac atg gtg gat gct cat ggc cca tcc tgt gcc agc tgg ctg tgt ccc 144
Asp Met Val Asp Ala His Gly Pro Ser Cys Ala Ser Trp Leu Cys Pro
        35                  40                  45

ttg ccc ctg gca ccg ggc agg tct gca ctg ctg gcc tgc cta cag gac 192
Leu Pro Leu Ala Pro Gly Arg Ser Ala Leu Leu Ala Cys Leu Gln Asp
    50                  55                  60

ctg gac ctg aac ctg tgc acc cca cag ccg gca ccc ctg ggc aca gac 240
Leu Asp Leu Asn Leu Cys Thr Pro Gln Pro Ala Pro Leu Gly Thr Asp
65                  70                  75                  80

ctg cag ggc ctc caa gag gac gcc ttg agc atg aag cac gag cca cca 288
Leu Gln Gly Leu Gln Glu Asp Ala Leu Ser Met Lys His Glu Pro Pro
                85                  90                  95

ggg ctg cag gcc agc tcc acc gat gac aag aaa ttc aca gtc aag tac 336
Gly Leu Gln Ala Ser Ser Thr Asp Asp Lys Lys Phe Thr Val Lys Tyr
            100                 105                 110

cca cag aac aag gac aag ctg gga aaa cag cca gaa aga gct ggc gag 384
Pro Gln Asn Lys Asp Lys Leu Gly Lys Gln Pro Glu Arg Ala Gly Glu
        115                 120                 125

ggg gcc ccc tgc cca gcc ttc tcc tct cat aac agc tct tcc cca cca 432
Gly Ala Pro Cys Pro Ala Phe Ser Ser His Asn Ser Ser Ser Pro Pro
    130                 135                 140

ccg ctg cag aac aga aag agc ccc agc ccc ttg gct ttc tgc ccc tgt 480
Pro Leu Gln Asn Arg Lys Ser Pro Ser Pro Leu Ala Phe Cys Pro Cys
145                 150                 155                 160

ccc cct gtc aac tcc atc tcc aag gag ctc cca ttt ctc ctc cac gcc 528
Pro Pro Val Asn Ser Ile Ser Lys Glu Leu Pro Phe Leu Leu His Ala
                165                 170                 175

ttc tac cct gga tac cca ctt ctc ctg cct cca ccc aac ctg ttc acc 576
Phe Tyr Pro Gly Tyr Pro Leu Leu Leu Pro Pro Pro Asn Leu Phe Thr
            180                 185                 190

tat ggg gcc cta cct tct gac caa tgt ccc cac ctc ctc atg ctg ccc 624
Tyr Gly Ala Leu Pro Ser Asp Gln Cys Pro His Leu Leu Met Leu Pro
        195                 200                 205

caa gac ccc tcc tac ccc acc atg gct atg cct agc ctg ctg atg atg 672
Gln Asp Pro Ser Tyr Pro Thr Met Ala Met Pro Ser Leu Leu Met Met
    210                 215                 220

gtc aat gag ctg ggg cac ccc agc gct cgg tgg gag acc ctg ctt ccc 720
Val Asn Glu Leu Gly His Pro Ser Ala Arg Trp Glu Thr Leu Leu Pro
225                 230                 235                 240

tac cca ggg gcc ttc caa gcc tct ggc caa gct ctg cct tcc cag gcc 768
Tyr Pro Gly Ala Phe Gln Ala Ser Gly Gln Ala Leu Pro Ser Gln Ala
                245                 250                 255

cga aat cca ggt gct gga gct gcc cca acc gac tcc cca ggc ctg gag 816
Arg Asn Pro Gly Ala Gly Ala Ala Pro Thr Asp Ser Pro Gly Leu Glu
            260                 265                 270

cgt ggt ggc atg gca tct cca gca aag cgg gtc cca ttg agt tcc cag 864
Arg Gly Gly Met Ala Ser Pro Ala Lys Arg Val Pro Leu Ser Ser Gln
        275                 280                 285

aca ggc acc gca gcc ttg cct tac ccg ctg aaa aag aag aat ggc aaa 912
Thr Gly Thr Ala Ala Leu Pro Tyr Pro Leu Lys Lys Lys Asn Gly Lys
    290                 295                 300

atc ctg tac gag tgc aac ata tgt ggc aag agc ttt ggg cag ctc tcc 960
Ile Leu Tyr Glu Cys Asn Ile Cys Gly Lys Ser Phe Gly Gln Leu Ser
305                 310                 315                 320

aat ctc aag gtc cac ctg cgt gtg cac agt gga gag cgt cca ttc cag 1008
Asn Leu Lys Val His Leu Arg Val His Ser Gly Glu Arg Pro Phe Gln
                325                 330                 335

tgt gcc ttg tgc cag aag agc ttc act caa ctt gcc cac ctg cag aag 1056
Cys Ala Leu Cys Gln Lys Ser Phe Thr Gln Leu Ala His Leu Gln Lys
            340                 345                 350

cac cac ctg gtg cac act ggg gag cgg ccc cac aag tgc tcg gtg tgc 1104
His His Leu Val His Thr Gly Glu Arg Pro His Lys Cys Ser Val Cys
        355                 360                 365

cac aag cgc ttc agc agc tcc agt aac ctc aag acc cac ctg cgc ctg 1152
His Lys Arg Phe Ser Ser Ser Ser Asn Leu Lys Thr His Leu Arg Leu
    370                 375                 380

cac tcc ggg gcc cgg ccc ttc cag tgc agt gtc tgc cgg agt cgc ttc 1200
His Ser Gly Ala Arg Pro Phe Gln Cys Ser Val Cys Arg Ser Arg Phe
385                 390                 395                 400

acc cag cac atc cac ctg aag ctg cac cat cgg ctg cat gcc cca cag 1248
Thr Gln His Ile His Leu Lys Leu His His Arg Leu His Ala Pro Gln
                405                 410                 415

ccc tgt ggc ctg gtg cac acc cag ctg ccc ctg gcc tct ctg gcc tgc 1296
Pro Cys Gly Leu Val His Thr Gln Leu Pro Leu Ala Ser Leu Ala Cys
            420                 425                 430

ctt gcc caa tgg cac cag ggg gca cta gat ctt atg gcg gtg gca tct 1344
Leu Ala Gln Trp His Gln Gly Ala Leu Asp Leu Met Ala Val Ala Ser
        435                 440                 445

gag aaa cac atg ggc tat gac ata gat gag gtc aaa gtg tcc tcg aca 1392
Glu Lys His Met Gly Tyr Asp Ile Asp Glu Val Lys Val Ser Ser Thr
    450                 455                 460

tcc cag ggg aaa gca aga gca gtg agc ctg agc agt gcc ggg act ccc 1440
Ser Gln Gly Lys Ala Arg Ala Val Ser Leu Ser Ser Ala Gly Thr Pro
465                 470                 475                 480

ctg gtg atg ggg cag gac cag aac aat taa 1470

Leu Val Met Gly Gln Asp Gln Asn Asn
                485

<210>  21
<211>  489
<212>  PRT
<213>  Homo sapiens

<400>  21

Met Ala Leu Gly Gly Thr Gly Gly Ser Leu Ser Pro Ser Leu Asp Phe
1               5                   10                  15

Gln Leu Phe Arg Gly Asp Gln Val Phe Ser Ala Cys Arg Pro Leu Pro
            20                  25                  30

Asp Met Val Asp Ala His Gly Pro Ser Cys Ala Ser Trp Leu Cys Pro
        35                  40                  45

Leu Pro Leu Ala Pro Gly Arg Ser Ala Leu Leu Ala Cys Leu Gln Asp
    50                  55                  60

Leu Asp Leu Asn Leu Cys Thr Pro Gln Pro Ala Pro Leu Gly Thr Asp
65                  70                  75                  80

Leu Gln Gly Leu Gln Glu Asp Ala Leu Ser Met Lys His Glu Pro Pro
                85                  90                  95

Gly Leu Gln Ala Ser Ser Thr Asp Asp Lys Lys Phe Thr Val Lys Tyr
            100                 105                 110

Pro Gln Asn Lys Asp Lys Leu Gly Lys Gln Pro Glu Arg Ala Gly Glu
        115                 120                 125

Gly Ala Pro Cys Pro Ala Phe Ser Ser His Asn Ser Ser Ser Pro Pro
    130                 135                 140

Pro Leu Gln Asn Arg Lys Ser Pro Ser Pro Leu Ala Phe Cys Pro Cys
145                 150                 155                 160

Pro Pro Val Asn Ser Ile Ser Lys Glu Leu Pro Phe Leu Leu His Ala
                165                 170                 175

Phe Tyr Pro Gly Tyr Pro Leu Leu Leu Pro Pro Pro Asn Leu Phe Thr
            180                 185                 190

Tyr Gly Ala Leu Pro Ser Asp Gln Cys Pro His Leu Leu Met Leu Pro
        195                 200                 205

Gln Asp Pro Ser Tyr Pro Thr Met Ala Met Pro Ser Leu Leu Met Met
    210                 215                 220

Val Asn Glu Leu Gly His Pro Ser Ala Arg Trp Glu Thr Leu Leu Pro
225                 230                 235                 240

Tyr Pro Gly Ala Phe Gln Ala Ser Gly Gln Ala Leu Pro Ser Gln Ala
                245                 250                 255

Arg Asn Pro Gly Ala Gly Ala Ala Pro Thr Asp Ser Pro Gly Leu Glu
            260                 265                 270

Arg Gly Gly Met Ala Ser Pro Ala Lys Arg Val Pro Leu Ser Ser Gln
        275                 280                 285

Thr Gly Thr Ala Ala Leu Pro Tyr Pro Leu Lys Lys Lys Asn Gly Lys
    290                 295                 300

Ile Leu Tyr Glu Cys Asn Ile Cys Gly Lys Ser Phe Gly Gln Leu Ser
305                 310                 315                 320

Asn Leu Lys Val His Leu Arg Val His Ser Gly Glu Arg Pro Phe Gln
                325                 330                 335

Cys Ala Leu Cys Gln Lys Ser Phe Thr Gln Leu Ala His Leu Gln Lys
            340                 345                 350

His His Leu Val His Thr Gly Glu Arg Pro His Lys Cys Ser Val Cys
        355                 360                 365

His Lys Arg Phe Ser Ser Ser Ser Asn Leu Lys Thr His Leu Arg Leu
    370                 375                 380

His Ser Gly Ala Arg Pro Phe Gln Cys Ser Val Cys Arg Ser Arg Phe
385                 390                 395                 400

Thr Gln His Ile His Leu Lys Leu His His Arg Leu His Ala Pro Gln
                405                 410                 415

Pro Cys Gly Leu Val His Thr Gln Leu Pro Leu Ala Ser Leu Ala Cys
            420                 425                 430

Leu Ala Gln Trp His Gln Gly Ala Leu Asp Leu Met Ala Val Ala Ser
        435                 440                 445

Glu Lys His Met Gly Tyr Asp Ile Asp Glu Val Lys Val Ser Ser Thr
    450                 455                 460

Ser Gln Gly Lys Ala Arg Ala Val Ser Leu Ser Ser Ala Gly Thr Pro
465                 470                 475                 480

Leu Val Met Gly Gln Asp Gln Asn Asn
                485

<210>  22
<211>  1404
<212>  DNA
<213>  Homo sapiens

<220>
<221>  CDS
<222>  (1)..(1404)

<400>  22
atg gcc ctg gga ggt aca ggg ggc tcc ctg tcc ccc agc ctg gac ttc      48
Met Ala Leu Gly Gly Thr Gly Gly Ser Leu Ser Pro Ser Leu Asp Phe
1               5                   10                  15

cag ctc ttc cga ggt gac cag gtc ttc tca gcc tgc aga cca ctt cca      96
Gln Leu Phe Arg Gly Asp Gln Val Phe Ser Ala Cys Arg Pro Leu Pro
            20                  25                  30

gac atg gtg gat gct cat ggc cca tcc tgt gcc agc tgg ctg tgt ccc      144
Asp Met Val Asp Ala His Gly Pro Ser Cys Ala Ser Trp Leu Cys Pro
        35                  40                  45

ttg ccc ctg gca ccg ggc agg tct gca ctg ctg gcc tgc cta cag gac      192
Leu Pro Leu Ala Pro Gly Arg Ser Ala Leu Leu Ala Cys Leu Gln Asp
    50                  55                  60

ctg gac ctg aac ctg tgc acc cca cag ccg gca ccc ctg ggc aca gac      240
Leu Asp Leu Asn Leu Cys Thr Pro Gln Pro Ala Pro Leu Gly Thr Asp
65                  70                  75                  80

ctg cag ggc ctc caa gag gac gcc ttg agc atg aag cac gag cca cca      288
Leu Gln Gly Leu Gln Glu Asp Ala Leu Ser Met Lys His Glu Pro Pro
                85                  90                  95

ggg ctg cag gcc agc tcc acc gat gac aag aaa ttc aca gtc aag tac      336
Gly Leu Gln Ala Ser Ser Thr Asp Asp Lys Lys Phe Thr Val Lys Tyr
            100                 105                 110

cca cag aac aag gac aag ctg gga aaa cag cca gaa aga gct ggc gag      384
Pro Gln Asn Lys Asp Lys Leu Gly Lys Gln Pro Glu Arg Ala Gly Glu
        115                 120                 125

ggg gcc ccc tgc cca gcc ttc tcc tct cat aac agc tct tcc cca cca      432
Gly Ala Pro Cys Pro Ala Phe Ser Ser His Asn Ser Ser Ser Pro Pro
    130                 135                 140

ccg ctg cag aac aga aag agc ccc agc ccc ttg gct ttc tgc ccc tgt      480

Pro Leu Gln Asn Arg Lys Ser Pro Ser Pro Leu Ala Phe Cys Pro Cys
145                 150                 155                 160

ccc cct gtc aac tcc atc tcc aag gag ctc cca ttt ctc ctc cac gcc      528
Pro Pro Val Asn Ser Ile Ser Lys Glu Leu Pro Phe Leu Leu His Ala
                165                 170                 175

ttc tac cct gga tac cca ctt ctc ctg cct cca ccc aac ctg ttc acc      576
Phe Tyr Pro Gly Tyr Pro Leu Leu Leu Pro Pro Pro Asn Leu Phe Thr
            180                 185                 190

tat ggg gcc cta cct tct gac caa tgt ccc cac ctc ctc atg ctg ccc      624
Tyr Gly Ala Leu Pro Ser Asp Gln Cys Pro His Leu Leu Met Leu Pro
        195                 200                 205

caa gac ccc tcc tac ccc acc atg gct atg cct agc ctg ctg atg atg      672
Gln Asp Pro Ser Tyr Pro Thr Met Ala Met Pro Ser Leu Leu Met Met
    210                 215                 220

gtc aat gag ctg ggg cac ccc agc gct cgg tgg gag acc ctg ctt ccc      720
Val Asn Glu Leu Gly His Pro Ser Ala Arg Trp Glu Thr Leu Leu Pro
225                 230                 235                 240

tac cca ggg gcc ttc caa gcc tct ggc caa gct ctg cct tcc cag gcc      768
Tyr Pro Gly Ala Phe Gln Ala Ser Gly Gln Ala Leu Pro Ser Gln Ala
                245                 250                 255

cga aat cca ggt gct gga gct gcc cca acc gac tcc cca ggc ctg gag      816
Arg Asn Pro Gly Ala Gly Ala Ala Pro Thr Asp Ser Pro Gly Leu Glu
            260                 265                 270

cgt ggt ggc atg gca tct cca gca aag cgg gtc cca ttg agt tcc cag      864
Arg Gly Gly Met Ala Ser Pro Ala Lys Arg Val Pro Leu Ser Ser Gln
        275                 280                 285

aca ggc acc gca gcc ttg cct tac ccg ctg aaa aag aag aat ggc aaa      912
Thr Gly Thr Ala Ala Leu Pro Tyr Pro Leu Lys Lys Lys Asn Gly Lys
    290                 295                 300

atc ctg tac gag tgc aac ata tgt ggc aag agc ttt ggg cag ctc tcc      960
Ile Leu Tyr Glu Cys Asn Ile Cys Gly Lys Ser Phe Gly Gln Leu Ser
305                 310                 315                 320

aat ctc aag gtc cac ctg cgt gtg cac agt gga gag cgt cca ttc cag     1008
Asn Leu Lys Val His Leu Arg Val His Ser Gly Glu Arg Pro Phe Gln
                325                 330                 335

tgt gcc ttg tgc cag aag agc ttc act caa ctt gcc cac ctg cag aag     1056
Cys Ala Leu Cys Gln Lys Ser Phe Thr Gln Leu Ala His Leu Gln Lys
            340                 345                 350

cac cac ctg gtg cac act ggg gag cgg ccc cac aag tgc tcg gtg tgc     1104
His His Leu Val His Thr Gly Glu Arg Pro His Lys Cys Ser Val Cys
        355                 360                 365

cac aag cgc ttc agc agc tcc agt aac ctc aag acc cac ctg cgc ctg     1152
His Lys Arg Phe Ser Ser Ser Ser Asn Leu Lys Thr His Leu Arg Leu
    370                 375                 380

cac tcc ggg gcc cgg ccc ttc cag tgc agt gtc tgc cgg agt cgc ttc     1200
His Ser Gly Ala Arg Pro Phe Gln Cys Ser Val Cys Arg Ser Arg Phe

385                 390                 395                 400

acc cag ctg ccc ctg gcc tct ctg gcc tgc ctt gcc caa tgg cac cag      1248
Thr Gln Leu Pro Leu Ala Ser Leu Ala Cys Leu Ala Gln Trp His Gln
                405                 410                 415

ggg gca cta gat ctt atg gcg gtg gca tct gag aaa cac atg ggc tat      1296
Gly Ala Leu Asp Leu Met Ala Val Ala Ser Glu Lys His Met Gly Tyr
            420                 425                 430

gac ata gat gag gtc aaa gtg tcc tcg aca tcc cag ggg aaa gca aga      1344
Asp Ile Asp Glu Val Lys Val Ser Ser Thr Ser Gln Gly Lys Ala Arg
        435                 440                 445

gca gtg agc ctg agc agt gcc ggg act ccc ctg gtg atg ggg cag gac      1392
Ala Val Ser Leu Ser Ser Ala Gly Thr Pro Leu Val Met Gly Gln Asp
    450                 455                 460

cag aac aat taa                                                      1404
Gln Asn Asn
465

<210>  23
<211>  467
<212>  PRT
<213>  Homo sapiens

<400>  23

Met Ala Leu Gly Gly Thr Gly Gly Ser Leu Ser Pro Ser Leu Asp Phe
1               5                   10                  15

Gln Leu Phe Arg Gly Asp Gln Val Phe Ser Ala Cys Arg Pro Leu Pro
            20                  25                  30

Asp Met Val Asp Ala His Gly Pro Ser Cys Ala Ser Trp Leu Cys Pro
        35                  40                  45

Leu Pro Leu Ala Pro Gly Arg Ser Ala Leu Leu Ala Cys Leu Gln Asp
    50                  55                  60

Leu Asp Leu Asn Leu Cys Thr Pro Gln Pro Ala Pro Leu Gly Thr Asp
65                  70                  75                  80

Leu Gln Gly Leu Gln Glu Asp Ala Leu Ser Met Lys His Glu Pro Pro
                85                  90                  95

Gly Leu Gln Ala Ser Ser Thr Asp Asp Lys Lys Phe Thr Val Lys Tyr
            100                 105                 110

Pro Gln Asn Lys Asp Lys Leu Gly Lys Gln Pro Glu Arg Ala Gly Glu
        115                 120                 125

Gly Ala Pro Cys Pro Ala Phe Ser Ser His Asn Ser Ser Ser Pro Pro
    130                 135                 140

Pro Leu Gln Asn Arg Lys Ser Pro Ser Pro Leu Ala Phe Cys Pro Cys
145                 150                 155                 160

Pro Pro Val Asn Ser Ile Ser Lys Glu Leu Pro Phe Leu Leu His Ala
                165                 170                 175

Phe Tyr Pro Gly Tyr Pro Leu Leu Leu Pro Pro Pro Asn Leu Phe Thr
            180                 185                 190

Tyr Gly Ala Leu Pro Ser Asp Gln Cys Pro His Leu Leu Met Leu Pro
        195                 200                 205

Gln Asp Pro Ser Tyr Pro Thr Met Ala Met Pro Ser Leu Leu Met Met
    210                 215                 220

Val Asn Glu Leu Gly His Pro Ser Ala Arg Trp Glu Thr Leu Leu Pro
225                 230                 235                 240

Tyr Pro Gly Ala Phe Gln Ala Ser Gly Gln Ala Leu Pro Ser Gln Ala
                245                 250                 255

Arg Asn Pro Gly Ala Gly Ala Ala Pro Thr Asp Ser Pro Gly Leu Glu
            260                 265                 270

Arg Gly Gly Met Ala Ser Pro Ala Lys Arg Val Pro Leu Ser Ser Gln
        275                 280                 285

Thr Gly Thr Ala Ala Leu Pro Tyr Pro Leu Lys Lys Lys Asn Gly Lys
    290                 295                 300

Ile Leu Tyr Glu Cys Asn Ile Cys Gly Lys Ser Phe Gly Gln Leu Ser
305                 310                 315                 320

Asn Leu Lys Val His Leu Arg Val His Ser Gly Glu Arg Pro Phe Gln
                325                 330                 335

Cys Ala Leu Cys Gln Lys Ser Phe Thr Gln Leu Ala His Leu Gln Lys
            340                 345                 350

His His Leu Val His Thr Gly Glu Arg Pro His Lys Cys Ser Val Cys
        355                 360                 365

His Lys Arg Phe Ser Ser Ser Ser Asn Leu Lys Thr His Leu Arg Leu
    370                 375                 380

His Ser Gly Ala Arg Pro Phe Gln Cys Ser Val Cys Arg Ser Arg Phe
385                 390                 395                 400

Thr Gln Leu Pro Leu Ala Ser Leu Ala Cys Leu Ala Gln Trp His Gln
                405                 410                 415

Gly Ala Leu Asp Leu Met Ala Val Ala Ser Glu Lys His Met Gly Tyr
            420                 425                 430

Asp Ile Asp Glu Val Lys Val Ser Ser Thr Ser Gln Gly Lys Ala Arg
        435                 440                 445

Ala Val Ser Leu Ser Ser Ala Gly Thr Pro Leu Val Met Gly Gln Asp
    450                 455                 460

Gln Asn Asn
465

<210>  24
<211>  1110
<212>  DNA
<213>  Homo sapiens

<220>
<221>  CDS
<222>  (1)..(1110)

<400>  24
atg gcc ctg gga ggt aca ggg ggc tcc ctg tcc ccc agc ctg gac ttc       48
Met Ala Leu Gly Gly Thr Gly Gly Ser Leu Ser Pro Ser Leu Asp Phe
1               5                   10                  15

cag ctc ttc cga ggt gac cag gtc ttc tca gcc tgc aga cca ctt cca       96
Gln Leu Phe Arg Gly Asp Gln Val Phe Ser Ala Cys Arg Pro Leu Pro
            20                  25                  30

gac atg gtg gat gct cat ggc cca tcc tgt gcc agc tgg ctg tgt ccc      144
Asp Met Val Asp Ala His Gly Pro Ser Cys Ala Ser Trp Leu Cys Pro
        35                  40                  45

ttg ccc ctg gca ccg ggc agg tct gca ctg ctg gcc tgc cta cag gac      192
Leu Pro Leu Ala Pro Gly Arg Ser Ala Leu Leu Ala Cys Leu Gln Asp
    50                  55                  60

ctg gac ctg aac ctg tgc acc cca cag ccg gca ccc ctg ggc aca gac      240
Leu Asp Leu Asn Leu Cys Thr Pro Gln Pro Ala Pro Leu Gly Thr Asp
65                  70                  75                  80

ctg cag ggc ctc caa gag gac gcc ttg agc atg aag cac gag cca cca      288
Leu Gln Gly Leu Gln Glu Asp Ala Leu Ser Met Lys His Glu Pro Pro

85                  90                  95

ggg ctg cag gcc agc tcc acc gat gac aag aaa ttc aca gtc aag tac        336
Gly Leu Gln Ala Ser Ser Thr Asp Asp Lys Lys Phe Thr Val Lys Tyr
            100                 105                 110

cca cag aac aag gac aag ctg gga aaa cag cca gaa aga gct ggc gag        384
Pro Gln Asn Lys Asp Lys Leu Gly Lys Gln Pro Glu Arg Ala Gly Glu
        115                 120                 125

ggg gcc ccc tgc cca gcc ttc tcc tct cat aac agc tct tcc cca cca        432
Gly Ala Pro Cys Pro Ala Phe Ser Ser His Asn Ser Ser Ser Pro Pro
    130                 135                 140

ccg ctg cag aac aga aag agc ccc agc ccc ttg gct ttc tgc ccc tgt        480
Pro Leu Gln Asn Arg Lys Ser Pro Ser Pro Leu Ala Phe Cys Pro Cys
145                 150                 155                 160

ccc cct gtc aac tcc atc tcc aag gag ctc cca ttt ctc ctc cac gcc        528
Pro Pro Val Asn Ser Ile Ser Lys Glu Leu Pro Phe Leu Leu His Ala
                165                 170                 175

ttc tac cct gga tac cca ctt ctc ctg cct cca ccc aac ctg ttc acc        576
Phe Tyr Pro Gly Tyr Pro Leu Leu Leu Pro Pro Pro Asn Leu Phe Thr
            180                 185                 190

tat ggg gcc cta cct tct gac caa tgt ccc cac ctc ctc atg ctg ccc        624
Tyr Gly Ala Leu Pro Ser Asp Gln Cys Pro His Leu Leu Met Leu Pro
        195                 200                 205

caa gac ccc tcc tac ccc acc atg gct atg cct agc ctg ctg atg atg        672
Gln Asp Pro Ser Tyr Pro Thr Met Ala Met Pro Ser Leu Leu Met Met
    210                 215                 220

gtc aat gag ctg ggg cac ccc agc gct cgg tgg gag acc ctg ctt ccc        720
Val Asn Glu Leu Gly His Pro Ser Ala Arg Trp Glu Thr Leu Leu Pro
225                 230                 235                 240

tac cca ggg gcc ttc caa gcc tct ggc caa gct ctg cct tcc cag gcc        768
Tyr Pro Gly Ala Phe Gln Ala Ser Gly Gln Ala Leu Pro Ser Gln Ala
                245                 250                 255

cga aat cca ggt gct gga gct gcc cca acc gac tcc cca ggc ctg gag        816
Arg Asn Pro Gly Ala Gly Ala Ala Pro Thr Asp Ser Pro Gly Leu Glu
            260                 265                 270

cgt ggt ggc atg gca tct cca gca aag cgg gtc cca ttg agt tcc cag        864
Arg Gly Gly Met Ala Ser Pro Ala Lys Arg Val Pro Leu Ser Ser Gln
        275                 280                 285

aca ggc acc gca gcc ttg cct tac ccg ctg aaa aag aag aat ggc aaa        912
Thr Gly Thr Ala Ala Leu Pro Tyr Pro Leu Lys Lys Lys Asn Gly Lys
    290                 295                 300

atc ctg tac gag tgc aac ata tgt ggc aag agc ttt ggg cag ctc tcc        960
Ile Leu Tyr Glu Cys Asn Ile Cys Gly Lys Ser Phe Gly Gln Leu Ser
305                 310                 315                 320

aat ctc aag tgg aga gcg tcc att cca gtg tgc ctt gtg cca gaa gag       1008
Asn Leu Lys Trp Arg Ala Ser Ile Pro Val Cys Leu Val Pro Glu Glu
                325                 330                 335

ctt cac tca act tgc cca cct gca gaa gca cca cct ggt gca cac tgg      1056
Leu His Ser Thr Cys Pro Pro Ala Glu Ala Pro Pro Gly Ala His Trp
            340                 345                 350

gga gcg gcc cca caa gtg ctc ggt gtg cca caa gcg ctt cag cag ctc      1104
Gly Ala Ala Pro Gln Val Leu Gly Val Pro Gln Ala Leu Gln Gln Leu
        355                 360                 365

cag taa                                                              1110
Gln

<210>  25
<211>  369
<212>  PRT
<213>  Homo sapiens

<400>  25

Met Ala Leu Gly Gly Thr Gly Gly Ser Leu Ser Pro Ser Leu Asp Phe
1               5                   10                  15

Gln Leu Phe Arg Gly Asp Gln Val Phe Ser Ala Cys Arg Pro Leu Pro
            20                  25                  30

Asp Met Val Asp Ala His Gly Pro Ser Cys Ala Ser Trp Leu Cys Pro
        35                  40                  45

Leu Pro Leu Ala Pro Gly Arg Ser Ala Leu Leu Ala Cys Leu Gln Asp
    50                  55                  60

Leu Asp Leu Asn Leu Cys Thr Pro Gln Pro Ala Pro Leu Gly Thr Asp
65                  70                  75                  80

Leu Gln Gly Leu Gln Glu Asp Ala Leu Ser Met Lys His Glu Pro Pro
                85                  90                  95

Gly Leu Gln Ala Ser Ser Thr Asp Asp Lys Lys Phe Thr Val Lys Tyr
            100                 105                 110

Pro Gln Asn Lys Asp Lys Leu Gly Lys Gln Pro Glu Arg Ala Gly Glu
        115                 120                 125

Gly Ala Pro Cys Pro Ala Phe Ser Ser His Asn Ser Ser Ser Pro Pro
    130                 135                 140

Pro Leu Gln Asn Arg Lys Ser Pro Ser Pro Leu Ala Phe Cys Pro Cys
145                 150                 155                 160

Pro Pro Val Asn Ser Ile Ser Lys Glu Leu Pro Phe Leu Leu His Ala
                165                 170                 175

Phe Tyr Pro Gly Tyr Pro Leu Leu Leu Pro Pro Pro Asn Leu Phe Thr
            180                 185                 190

Tyr Gly Ala Leu Pro Ser Asp Gln Cys Pro His Leu Leu Met Leu Pro
        195                 200                 205

Gln Asp Pro Ser Tyr Pro Thr Met Ala Met Pro Ser Leu Leu Met Met
    210                 215                 220

Val Asn Glu Leu Gly His Pro Ser Ala Arg Trp Glu Thr Leu Leu Pro
225                 230                 235                 240

Tyr Pro Gly Ala Phe Gln Ala Ser Gly Gln Ala Leu Pro Ser Gln Ala
                245                 250                 255

Arg Asn Pro Gly Ala Gly Ala Ala Pro Thr Asp Ser Pro Gly Leu Glu
            260                 265                 270

Arg Gly Gly Met Ala Ser Pro Ala Lys Arg Val Pro Leu Ser Ser Gln
        275                 280                 285

Thr Gly Thr Ala Ala Leu Pro Tyr Pro Leu Lys Lys Lys Asn Gly Lys
    290                 295                 300

Ile Leu Tyr Glu Cys Asn Ile Cys Gly Lys Ser Phe Gly Gln Leu Ser
305                 310                 315                 320

Asn Leu Lys Trp Arg Ala Ser Ile Pro Val Cys Leu Val Pro Glu Glu
                325                 330                 335

Leu His Ser Thr Cys Pro Pro Ala Glu Ala Pro Pro Gly Ala His Trp
            340                 345                 350

Gly Ala Ala Pro Gln Val Leu Gly Val Pro Gln Ala Leu Gln Gln Leu
        355                 360                 365

Gln

<210>  26
<211>  903
<212>  DNA
<213>  Homo sapiens

<220>
<221> CDS
<222> (1)..(903)

<400> 26
atg gcc ctg gga ggt aca ggg ggc tcc ctg tcc ccc agc ctg gac ttc 48
Met Ala Leu Gly Gly Thr Gly Gly Ser Leu Ser Pro Ser Leu Asp Phe
1               5                   10                  15

cag ctc ttc cga ggt gac cag gtc ttc tca gcc tgc aga cca ctt cca 96
Gln Leu Phe Arg Gly Asp Gln Val Phe Ser Ala Cys Arg Pro Leu Pro
            20                  25                  30

gac atg gtg gat gct cat ggc cca tcc tgt gcc agc tgg ctg tgt ccc 144
Asp Met Val Asp Ala His Gly Pro Ser Cys Ala Ser Trp Leu Cys Pro
        35                  40                  45

ttg ccc ctg gca ccg ggc agg tct gca ctg ctg gcc tgc cta cag gac 192
Leu Pro Leu Ala Pro Gly Arg Ser Ala Leu Leu Ala Cys Leu Gln Asp
    50                  55                  60

ctg gac ctg aac ctg tgc acc cca cag ccg gca ccc ctg ggc aca gac 240
Leu Asp Leu Asn Leu Cys Thr Pro Gln Pro Ala Pro Leu Gly Thr Asp
65                  70                  75                  80

ctg cag ggc ctc caa gag gac gcc ttg agc atg aag cac gag cca cca 288
Leu Gln Gly Leu Gln Glu Asp Ala Leu Ser Met Lys His Glu Pro Pro
                85                  90                  95

ggg ctg cag gcc agc tcc acc gat gac aag aaa ttc aca gtc aag tac 336
Gly Leu Gln Ala Ser Ser Thr Asp Asp Lys Lys Phe Thr Val Lys Tyr
            100                 105                 110

cca ctt ctc ctg cct cca ccc aac ctg ttc acc tat ggg gcc cta cct 384
Pro Leu Leu Leu Pro Pro Pro Asn Leu Phe Thr Tyr Gly Ala Leu Pro
        115                 120                 125

tct gac caa tgt ccc cac ctc ctc atg ctg ccc caa gac ccc tcc tac 432
Ser Asp Gln Cys Pro His Leu Leu Met Leu Pro Gln Asp Pro Ser Tyr
    130                 135                 140

ccc acc atg gct atg cct agc ctg ctg atg atg gtc aat gag ctg ggg 480
Pro Thr Met Ala Met Pro Ser Leu Leu Met Met Val Asn Glu Leu Gly
145                 150                 155                 160

cac ccc agc gct cgg tgg gag acc ctg ctt ccc tac cca ggg gcc ttc 528
His Pro Ser Ala Arg Trp Glu Thr Leu Leu Pro Tyr Pro Gly Ala Phe
                165                 170                 175

caa gcc tct ggc caa gct ctg cct tcc cag gcc cga aat cca ggt gct 576
Gln Ala Ser Gly Gln Ala Leu Pro Ser Gln Ala Arg Asn Pro Gly Ala
            180                 185                 190

gga gct gcc cca acc gac tcc cca ggc ctg gag cgt ggt ggc atg gca 624
Gly Ala Ala Pro Thr Asp Ser Pro Gly Leu Glu Arg Gly Gly Met Ala
        195                 200                 205

tct cca gca aag cgg gtc cca ttg agt tcc cag aca ggc acc gca gcc 672
Ser Pro Ala Lys Arg Val Pro Leu Ser Ser Gln Thr Gly Thr Ala Ala
    210                 215                 220

ttg cct tac ccg ctg aaa aag aag aat ggc aaa atc ctg tac gag tgc 720
Leu Pro Tyr Pro Leu Lys Lys Lys Asn Gly Lys Ile Leu Tyr Glu Cys
225                 230                 235                 240

aac ata tgt ggc aag agc ttt ggg cag ctc tcc aat ctc aag tgg aga 768
Asn Ile Cys Gly Lys Ser Phe Gly Gln Leu Ser Asn Leu Lys Trp Arg
                245                 250                 255

gcg tcc att cca gtg tgc ctt gtg cca gaa gag ctt cac tca act tgc 816
Ala Ser Ile Pro Val Cys Leu Val Pro Glu Glu Leu His Ser Thr Cys
            260                 265                 270

cca cct gca gaa gca cca cct ggt gca cac tgg gga gcg gcc cca caa 864
Pro Pro Ala Glu Ala Pro Pro Gly Ala His Trp Gly Ala Ala Pro Gln
        275                 280                 285

gtg ctc ggt gtg cca caa gcg ctt cag cag ctc cag taa 903
Val Leu Gly Val Pro Gln Ala Leu Gln Gln Leu Gln
    290                 295                 300

<210> 27
<211> 300
<212> PRT
<213> Homo sapiens

<400> 27

Met Ala Leu Gly Gly Thr Gly Gly Ser Leu Ser Pro Ser Leu Asp Phe
1               5                   10                  15

Gln Leu Phe Arg Gly Asp Gln Val Phe Ser Ala Cys Arg Pro Leu Pro
            20                  25                  30

Asp Met Val Asp Ala His Gly Pro Ser Cys Ala Ser Trp Leu Cys Pro
        35                  40                  45

Leu Pro Leu Ala Pro Gly Arg Ser Ala Leu Leu Ala Cys Leu Gln Asp
    50                  55                  60

Leu Asp Leu Asn Leu Cys Thr Pro Gln Pro Ala Pro Leu Gly Thr Asp
65                  70                  75                  80

Leu Gln Gly Leu Gln Glu Asp Ala Leu Ser Met Lys His Glu Pro Pro
                85                  90                  95

Gly Leu Gln Ala Ser Ser Thr Asp Asp Lys Lys Phe Thr Val Lys Tyr
            100                 105                 110

Pro Leu Leu Leu Pro Pro Pro Asn Leu Phe Thr Tyr Gly Ala Leu Pro
        115                 120                 125

Ser Asp Gln Cys Pro His Leu Leu Met Leu Pro Gln Asp Pro Ser Tyr
    130                 135                 140

Pro Thr Met Ala Met Pro Ser Leu Leu Met Met Val Asn Glu Leu Gly
145                 150                 155                 160

His Pro Ser Ala Arg Trp Glu Thr Leu Leu Pro Tyr Pro Gly Ala Phe
                165                 170                 175

Gln Ala Ser Gly Gln Ala Leu Pro Ser Gln Ala Arg Asn Pro Gly Ala
            180                 185                 190

Gly Ala Ala Pro Thr Asp Ser Pro Gly Leu Glu Arg Gly Gly Met Ala
        195                 200                 205

Ser Pro Ala Lys Arg Val Pro Leu Ser Ser Gln Thr Gly Thr Ala Ala
    210                 215                 220

Leu Pro Tyr Pro Leu Lys Lys Lys Asn Gly Lys Ile Leu Tyr Glu Cys
225                 230                 235                 240

Asn Ile Cys Gly Lys Ser Phe Gly Gln Leu Ser Asn Leu Lys Trp Arg
                245                 250                 255

Ala Ser Ile Pro Val Cys Leu Val Pro Glu Glu Leu His Ser Thr Cys
            260                 265                 270

Pro Pro Ala Glu Ala Pro Pro Gly Ala His Trp Gly Ala Ala Pro Gln
        275                 280                 285

Val Leu Gly Val Pro Gln Ala Leu Gln Gln Leu Gln
    290                 295                 300

<210>  28
<211>  1725
<212>  DNA
<213>  Homo sapiens

<400>  28
ggggaggtag cacaaaggga ggaccatggt cttctaactg aggaacctga gcagatgcct      60

tgacccagac cagagaccca agccaaggaa agcccatgat caccagagta atggggatat     120

gaaggaagaa tcagctgcac aattaggttg ttgtcatagg cccatggccc tgggaggtac     180

agggggctcc ctgtccccca gcctggactt ccagctcttc cgaggtgacc aggtcttctc     240

agcctgcaga ccacttccag acatggtgga tgctcatggc ccatcctgtg ccagctggct     300

gtgtcccttg cccctggcac cgggcaggtc tgcactgctg gcctgcctac aggacctgga     360

cctgaacctg tgcaccccac agccggcacc cctgggcaca gacctgcagg gcctccaaga 420

ggacgccttg agcatgaagc acgagccacc agggctgcag gccagctcca ccgatgacaa 480

gaaattcaca gtcaagtacc cacagaacaa ggacaagctg ggaaaacagc cagaaagagc 540

tggcgagggg gccccctgcc cagccttctc ctctcataac agctcttccc caccaccgct 600

gcagaacaga aagagcccca gccccttggc tttctgcccc tgtccccctg tcaactccat 660

ctccaaggag ctcccatttc tcctccacgc cttctaccct ggatacccac ttctcctgcc 720

tccacccaac ctgttcacct atggggccct accttctgac caatgtcccc acctcctcat 780

gctgccccaa gacccctcct accccaccat ggctatgcct agcctgctga tgatggtcaa 840

tgagctgggg caccccagcg ctcggtggga gaccctgctt ccctacccag gggccttcca 900

agcctctggc caagctctgc cttcccaggc ccgaaatcca ggtgctggag ctgccccaac 960

cgactcccca ggcctggagc gtggtggcat ggcatctcca gcaaagcggg tcccattgag 1020

ttcccagaca ggcaccgcag ccttgcctta cccgctgaaa aagaagaatg gcaaaatcct 1080

gtacgagtgc aacatatgtg gcaagagctt tgggcagctc tccaatctca aggtccacct 1140

gcgtgtgcac agtggagagc gtccattcca gtgtgccttg tgccagaaga gcttcactca 1200

acttgcccac ctgcagaagc accacctggt gcacactggg gagcggcccc acaagtgctc 1260

gataccgtgg gttccaggaa gaaatcactg gaaaagcttc caggcctgga gggaaagaga 1320

ggtgtgccac aagcgcttca gcagctccag taacctcaag acccacctgc gcctgcactc 1380

cggggcccgg cccttccagt gcagtgtctg ccggagtcgc ttcacccagc acatccacct 1440

gaagctgcac catcggctgc atgccccaca gccctgtggc ctggtgcaca cccagctgcc 1500

cctggcctct ctggcctgcc ttgcccaatg gcaccagggg gcactagatc ttatggcggt 1560

ggcatctgag aaacacatgg gctatgacat agatgaggtc aaagtgtcct cgacatccca 1620

ggggaaagca agagcagtga gcctgagcag tgccgggact cccctggtga tggggcagga 1680

ccagaacaat taaaaatgtt cttctgtcaa aaaaaaaaaa aaaat 1725

<210> 29
<211> 1725
<212> DNA
<213> Homo sapiens

<400> 29
ggggaggtag cacaaaggga ggaccatggt cttctaactg aggaacctga gcagatgcct 60

tgacccagac cagagaccca agccaaggaa agcccatgat caccagagta atggggatat 120

gaaggaagaa tcagctgcac aattaggttg ttgtcatagg cccatggccc tgggaggtac 180

agggggctcc ctgtccccca gcctggactt ccagctcttc cgaggtgacc aggtcttctc 240

agcctgcaga ccacttccag acatggtgga tgctcatggc ccatcctgtg ccagctggct 300

gtgtcccttg cccctggcac cgggcaggtc tgcactgctg gcctgcctac aggacctgga 360

cctgaacctg tgcaccccac agccggcacc cctgggcaca gacctgcagg gcctccaaga 420

ggacgccttg agcatgaagc acgagccacc agggctgcag gccagctcca ccgatgacaa 480

gaaattcaca gtcaagtacc cacagaacaa ggacaagctg ggaaaacagc cagaaagagc 540

tggcgagggg gccccctgcc cagccttctc ctctcataac agctcttccc caccaccgct 600

gcagaacaga aagagcccca gccccttggc tttctgcccc tgtccccctg tcaactccat 660

ctccaaggag ctcccatttc tcctccacgc cttctaccct ggatacccac ttctcctgcc 720

tccacccaac ctgttcacct atggggccct accttctgac caatgtcccc acctcctcat 780

gctgccccaa gacccctcct accccaccat ggctatgcct agcctgctga tgatggtcaa 840

tgagctgggg caccccagcg ctcggtggga gaccctgctt ccctacccag gggccttcca 900

agcctctggc caagctctgc cttcccaggc ccgaaatcca ggtgctggag ctgccccaac 960

cgactcccca ggcctggagc gtggtggcat ggcatctcca gcaaagcggg tcccattgag 1020

ttcccagaca ggcaccgcag ccttgcctta cccgctgaaa aagaagaatg gcaaaatcct 1080

gtacgagtgc aacatatgtg gcaagagctt tgggcagctc tccaatctca aggtccacct 1140

gcgtgtgcac agtggagagc gtccattcca gtgtgccttg tgccagaaga gcttcactca 1200

acttgcccac ctgcagaagc accacctggt gcacactggg gagcggcccc acaagtgctc 1260

gataccgtgg gttccaggaa gaaatcactg gaaaagcttc caggcctgga gggaaagaga 1320

ggtgtgccac aagcgcttca gcagctccag taacctcaag acccacctgc gcctgcactc 1380

cggggcccgg cccttccagt gcagtgtctg ccggagtcgc ttcacccagc acatccacct 1440

gaagctgcac catcggctgc atgccccaca gccctgtggc ctggtgcaca cccagctgcc 1500

cctggcctct ctggcctgcc ttgcccaatg gcaccagggg gcactagatc ttatggcggt 1560

ggcatctgag aaacacatgg gctatgacat agatgaggtc aaagtgtcct cgacatccca 1620

ggggaaagca agagcagtga gcctgagcag tgccgggact cccctggtga tggggcagga 1680

ccagaacaat taaaaatgtt tcttctgtca aaaaaaaaaa aaaaa 1725

<210> 30
<211> 1665
<212> DNA
<213> Homo sapiens

<400> 30
ggggaggtag cacaaaggga ggaccatggt cttctaactg aggaacctga gcagatgcct 60

tgacccagac cagagaccca agccaaggaa agcccatgat caccagagta atggggatat 120

gaaggaagaa tcagctgcac aattaggttg ttgtcatagg cccatggccc tgggaggtac 180

agggggctcc ctgtccccca gcctggactt ccagctcttc cgaggtgacc aggtcttctc 240

agcctgcaga ccacttccag acatggtgga tgctcatggc ccatcctgtg ccagctggct 300

gtgtcccttg cccctggcac cgggcaggtc tgcactgctg gcctgcctac aggacctgga 360

cctgaacctg tgcaccccac agccggcacc cctgggcaca gacctgcagg gcctccaaga 420

ggacgccttg agcatgaagc acgagccacc agggctgcag gccagctcca ccgatgacaa 480

gaaattcaca gtcaagtacc cacagaacaa ggacaagctg ggaaaacagc cagaaagagc 540

tggcgagggg gccccctgcc cagccttctc ctctcataac agctcttccc caccaccgct 600

gcagaacaga aagagcccca gccccttggc tttctgcccc tgtccccctg tcaactccat 660

ctccaaggag ctcccatttc tcctccacgc cttctaccct ggatacccac ttctcctgcc 720

tccacccaac ctgttcacct atggggccct accttctgac caatgtcccc acctcctcat 780

gctgccccaa gacccctcct accccaccat ggctatgcct agcctgctga tgatggtcaa 840

tgagctgggg caccccagcg ctcggtggga gaccctgctt ccctacccag gggccttcca 900

agcctctggc caagctctgc cttcccaggc ccgaaatcca ggtgctggag ctgccccaac 960

cgactcccca ggcctggagc gtggtggcat ggcatctcca gcaaagcggg tcccattgag 1020

ttcccagaca ggcaccgcag ccttgcctta cccgctgaaa aagaagaatg gcaaaatcct 1080

gtacgagtgc aacatatgtg gcaagagctt tgggcagctc tccaatctca aggtccacct 1140

gcgtgtgcac agtggagagc gtccattcca gtgtgccttg tgccagaaga gcttcactca 1200

acttgcccac ctgcagaagc accacctggt gcacactggg gagcggcccc acaagtgctc 1260

ggtgtgccac aagcgcttca gcagctccag taacctcaag acccacctgc gcctgcactc 1320

cggggcccgg cccttccagt gcagtgtctg ccggagtcgc ttcacccagc acatccacct 1380

gaagctgcac catcggctgc atgccccaca gccctgtggc ctggtgcaca cccagctgcc 1440

cctggcctct ctggcctgcc ttgcccaatg gcaccagggg gcactagatc ttatggcggt 1500

ggcatctgag aaacacatgg gctatgacat agatgaggtc aaagtgtcct cgacatccca 1560

ggggaaagca agagcagtga gcctgagcag tgccgggact cccctggtga tggggcagga 1620

ccagaacaat taaaaatgtt tcttctgtca aaaaaaaaaa aaaaa 1665

<210> 31
<211> 1599
<212> DNA
<213> Homo sapiens

<400> 31
ggggaggtag cacaaaggga ggaccatggt cttctaactg aggaacctga gcagatgcct 60

tgacccagac cagagaccca agccaaggaa agcccatgat caccagagta atggggatat 120

gaaggaagaa tcagctgcac aattaggttg ttgtcatagg cccatggccc tgggaggtac 180

agggggctcc ctgtccccca gcctggactt ccagctcttc cgaggtgacc aggtcttctc 240

agcctgcaga ccacttccag acatggtgga tgctcatggc ccatcctgtg ccagctggct 300

gtgtcccttg cccctggcac cgggcaggtc tgcactgctg gcctgcctac aggacctgga 360

cctgaacctg tgcaccccac agccggcacc cctgggcaca gacctgcagg gcctccaaga 420

ggacgccttg agcatgaagc acgagccacc agggctgcag gccagctcca ccgatgacaa 480

gaaattcaca gtcaagtacc cacagaacaa ggacaagctg ggaaaacagc cagaaagagc 540

tggcgagggg gccccctgcc cagccttctc ctctcataac agctcttccc caccaccgct 600

gcagaacaga aagagcccca gccccttggc tttctgcccc tgtccccctg tcaactccat 660

ctccaaggag ctcccatttc tcctccacgc cttctaccct ggatacccac ttctcctgcc 720

tccacccaac ctgttcacct atggggccct accttctgac caatgtcccc acctcctcat 780

gctgccccaa gacccctcct accccaccat ggctatgcct agcctgctga tgatggtcaa 840

tgagctgggg caccccagcg ctcggtggga gaccctgctt ccctacccag gggccttcca 900

agcctctggc caagctctgc cttcccaggc ccgaaatcca ggtgctggag ctgccccaac 960

cgactcccca ggcctggagc gtggtggcat ggcatctcca gcaaagcggg tcccattgag 1020

ttcccagaca ggcaccgcag ccttgcctta cccgctgaaa aagaagaatg gcaaaatcct 1080

gtacgagtgc aacatatgtg gcaagagctt tgggcagctc tccaatctca aggtccacct 1140

gcgtgtgcac agtggagagc gtccattcca gtgtgccttg tgccagaaga gcttcactca 1200

acttgcccac ctgcagaagc accacctggt gcacactggg gagcggcccc acaagtgctc 1260

ggtgtgccac aagcgcttca gcagctccag taacctcaag acccacctgc gcctgcactc 1320

cggggcccgg cccttccagt gcagtgtctg ccggagtcgc ttcacccagc tgcccctggc 1380

ctctctggcc tgccttgccc aatggcacca gggggcacta gatcttatgg cggtggcatc 1440

tgagaaacac atgggctatg acatagatga ggtcaaagtg tcctcgacat cccaggggaa 1500

agcaagagca gtgagcctga gcagtgccgg gactcccctg gtgatggggc aggaccagaa 1560

caattaaaaa tgtttcttct gtcaaaaaaa aaaaaaaaa 1599

<210> 32
<211> 1645
<212> DNA
<213> Homo sapiens

<400> 32
ggggaggtag cacaaaggga ggaccatggt cttctaactg aggaacctga gcagatgcct 60

tgacccagac cagagaccca agccaaggaa agcccatgat caccagagta atggggatat 120

gaaggaagaa tcagctgcac aattaggttg ttgtcatagg cccatggccc tgggaggtac 180

agggggctcc ctgtccccca gcctggactt ccagctcttc cgaggtgacc aggtcttctc 240

agcctgcaga ccacttccag acatggtgga tgctcatggc ccatcctgtg ccagctggct 300

gtgtcccttg cccctggcac cgggcaggtc tgcactgctg gcctgcctac aggacctgga 360

cctgaacctg tgcaccccac agccggcacc cctgggcaca gacctgcagg gcctccaaga 420

ggacgccttg agcatgaagc acgagccacc agggctgcag gccagctcca ccgatgacaa 480

gaaattcaca gtcaagtacc cacagaacaa ggacaagctg ggaaaacagc cagaaagagc 540

tggcgagggg gccccctgcc cagccttctc ctctcataac agctcttccc caccaccgct 600

gcagaacaga aagagcccca gccccttggc tttctgcccc tgtccccctg tcaactccat 660

ctccaaggag ctcccatttc tcctccacgc cttctaccct ggatacccac ttctcctgcc 720

tccacccaac ctgttcacct atggggccct accttctgac caatgtcccc acctcctcat 780

gctgccccaa gacccctcct accccaccat ggctatgcct agcctgctga tgatggtcaa 840

tgagctgggg caccccagcg ctcggtggga gaccctgctt ccctacccag gggccttcca 900

agcctctggc caagctctgc cttcccaggc ccgaaatcca ggtgctggag ctgccccaac 960

cgactcccca ggcctggagc gtggtggcat ggcatctcca gcaaagcggg tcccattgag 1020

ttcccagaca ggcaccgcag ccttgcctta cccgctgaaa aagaagaatg gcaaaatcct 1080

gtacgagtgc aacatatgtg gcaagagctt tgggcagctc tccaatctca agtggagagc 1140

gtccattcca gtgtgccttg tgccagaaga gcttcactca acttgcccac ctgcagaagc 1200

accacctggt gcacactggg gagcggcccc acaagtgctc ggtgtgccac aagcgcttca 1260

gcagctccag taacctcaag acccacctgc gcctgcactc cggggcccgg cccttccagt 1320

gcagtgtctg ccggagtcgc ttcacccagc acatccacct gaagctgcac catcggctgc 1380

atgccccaca gccctgtggc ctggtgcaca cccagctgcc cctggcctct ctggcctgcc 1440

ttgcccaatg gcaccagggg gcactagatc ttatggcggt ggcatctgag aaacacatgg 1500

gctatgacat agatgaggtc aaagtgtcct cgacatccca ggggaaagca agagcagtga 1560

gcctgagcag tgccgggact cccctggtga tggggcagga ccagaacaat taaaaatgtt 1620

tcttctgtca aaaaaaaaaa aaaaa 1645

<210> 33
<211> 1438
<212> DNA
<213> Homo sapiens

<400> 33
   ggggaggtag cacaaaggga ggaccatggt cttctaactg aggaacctga gcagatgcct 60
   tgacccagac cagagaccca agccaaggaa agcccatgat caccagagta atggggatat 120
   gaaggaagaa tcagctgcac aattaggttg ttgtcatagg cccatggccc tgggaggtac 180
   agggggctcc ctgtccccca gcctggactt ccagctcttc cgaggtgacc aggtcttctc 240
   agcctgcaga ccacttccag acatggtgga tgctcatggc ccatcctgtg ccagctggct 300
   gtgtcccttg cccctggcac cgggcaggtc tgcactgctg gcctgcctac aggacctgga 360
   cctgaacctg tgcaccccac agccggcacc cctgggcaca gacctgcagg gcctccaaga 420
   ggacgccttg agcatgaagc acgagccacc agggctgcag gccagctcca ccgatgacaa 480
   gaaattcaca gtcaagtacc cacttctcct gcctccaccc aacctgttca cctatggggc 540
   cctaccttct gaccaatgtc cccacctcct catgctgccc caagacccct cctaccccac 600
   catggctatg cctagcctgc tgatgatggt caatgagctg gggcacccca gcgctcggtg 660
   ggagaccctg cttccctacc caggggcctt ccaagcctct ggccaagctc tgccttccca 720
   ggcccgaaat ccaggtgctg gagctgcccc aaccgactcc ccaggcctgg agcgtggtgg 780
   catggcatct ccagcaaagc gggtcccatt gagttcccag acaggcaccg cagccttgcc 840
   ttacccgctg aaaaagaaga atggcaaaat cctgtacgag tgcaacatat gtggcaagag 900
   ctttgggcag ctctccaatc tcaagtggag agcgtccatt ccagtgtgcc ttgtgccaga 960
   agagcttcac tcaacttgcc cacctgcaga agcaccacct ggtgcacact ggggagcggc 1020
   cccacaagtg ctcggtgtgc cacaagcgct tcagcagctc cagtaacctc aagacccacc 1080
   tgcgcctgca ctccggggcc cggcccttcc agtgcagtgt ctgccggagt cgcttcaccc 1140
   agcacatcca cctgaagctg caccatcggc tgcatgcccc acagccctgt ggcctggtgc 1200
   acacccagct gcccctggcc tctctggcct gccttgccca atggcaccag ggggcactag 1260
   atcttatggc ggtggcatct gagaaacaca tgggctatga catagatgag gtcaaagtgt 1320
   cctcgacatc ccaggggaaa gcaagagcag tgagcctgag cagtgccggg actcccctgg 1380
   tgatggggca ggaccagaac aattaaaaat gtttcttctg tcaaaaaaaa aaaaaaaa 1438

<210> 34
<211> 1725
<212> DNA
<213> Homo sapiens

<400> 34
ggggaggtag cacaaaggga ggaccatggt cttctaactg aggaacctga gcagatgcct 60

tgacccagac cagagaccca agccaaggaa agcccatgat caccagagta atggggatat 120

gaaggaagaa tcagctgcac aattaggttg ttgtcatagg cccatggccc tgggaggtac 180

agggggctcc ctgtccccca gcctggactt ccagctcttc cgaggtgacc aggtcttctc 240
agcctgcaga ccacttccag acatggtgga tgctcatggc ccatcctgtg ccagctggct 300
gtgtcccttg cccctggcac cgggcaggtc tgcactgctg gcctgcctac aggacctgga 360
cctgaacctg tgcaccccac agccggcacc cctgggcaca gacctgcagg gcctccaaga 420
ggacgccttg agcatgaagc acgagccacc agggctgcag gccagctcca ccgatgacaa 480
gaaattcaca gtcaagtacc cacagaacaa ggacaagctg ggaaaacagc cagaaagagc 540
tggcgagggg gccccctgcc cagccttctc ctctcataac agctcttccc caccaccgct 600
gcagaacaga aagagcccca gccccttggc tttctgcccc tgtccccctg tcaactccat 660
ctccaaggag ctcccatttc tcctccacgc cttctaccct ggatacccac ttctcctgcc 720
tccacccaac ctgttcacct atggggccct accttctgac caatgtcccc acctcctcat 780
gctgccccaa gacccctcct accccaccat ggctatgcct agcctgctga tgatggtcaa 840
tgagctgggg caccccagcg ctcggtggga gaccctgctt ccctacccag gggccttcca 900
agcctctggc caagctctgc cttcccaggc ccgaaatcca ggtgctggag ctgccccaac 960
cgactcccca ggcctggagc gtggtggcat ggcatctcca gcaaagcggg tcccattgag 1020
ttcccagaca ggcaccgcag ccttgcctta cccgctgaaa aagaagaatg gcaaaatcct 1080
gtacgagtgc aacatatgtg gcaagagctt tgggcagctc tccaatctca aggtccacct 1140
gcgtgtgcac agtggagagc gtccattcca gtgtgccttg tgccagaaga gcttcactca 1200
acttgcccac ctgcagaagc accacctggt gcacactggg gagcggcccc acaagtgctc 1260
gataccgtgg gttccaggaa gaaatcactg gaaaagcttc caggcctgga gggaaagaga 1320
ggtgtgccac aagcgcttca gcagctccag taacctcaag acccacctgc gcctgcactc 1380
cggggcccgg cccttccagt gcagtgtctg ccggagtcgc ttcacccagc acatccacct 1440
gaagctgcac catcggctgc atgccccaca gccctgtggc ctggtgcaca cccagctgcc 1500
cctggcctct ctggcctgcc ttgcccaatg gcaccagggg gcactagatc ttatggcggt 1560
ggcatctgag aaacacatgg gctatgacat agatgaggtc aaagtgtcct cgacatccca 1620
ggggaaagca agagcagtga gcctgagcag tgccgggact cccctggtga tggggcagga 1680
ccagaacaat taaaaatgtt tcttctgtca aaaaaaaaaa aaaaa 1725

<210> 35
<211> 1387
<212> DNA
<213> Mus musculus

<400> 35
ttgctacaag atgaaagccc tggatggttt aagagagtcc ctgtacccca gtctggactt 60

ccagctctat caagatgacc aggtctgctc agctgacgct tctcagccac ttgcagactc 120

agtgggtgct catgacttag cctggagcga gaggatgtgc cccttaccac tggcaccagc 180

taaatctccc ttgctggcct gccccgaaag cccggaccta tgcttgtgtg ccctgcagaa 240

gacaccccta ggcagagccc cacaggacct cggagaagac gcctcaaaca tgagacacca 300

gccgccaagc ttgtacaaag cctccaccga cagtgagaaa ctcacaatca aggactcact 360

gaacagagag gagatgggaa atgagccaga aagaggcgcc tacccacacc tccctccccg 420

tactagctcc ttcccagatg ctgggctgga cagaaagagc cttagtccct tgaccttctg 480

gccctggctt cctcccactc tcatctccaa ggaaccccct atccacatct accccatctt 540

ccctgggtac ccacttctgc ctctccctta cctattcact tatggggccc taccttctgc 600

ccaacatcca tacctcttca tgctgccccc acactccaca taccccaccg tggctgggcc 660

cagcttgctt atgacagcca gtgggtctgg acctcgtatt ccccaagaga agaccctgct 720

tctccactcg ggagctttcc agagtgccgg ccacacccta cactcccagg ttgagagtcg 780

gagctctaga gatacccgga ccccaggaca agctggtgtg gcagctccaa caaggcgggc 840

tgtgccaggc tcccgggcag gtgtcatagc cctgccctac cctctgaaga aagagaatgg 900

taaaatctta tacgagtgta acgtgtgtgg caagaacttt gggcaactct ccaacctcaa 960

ggttcacctg cgagtacaca gtggcgagcg cccgttccag tgtgccctgt gtcagaagcg 1020

cttcacccag ctggcccacc tgcaaaagca ccacctggtg cacaccgggg agcggcccca 1080

ccagtgccag gtgtgccaca agcgcttcag tagctccagc aacctcaaga cccatcttcg 1140

gctgcactca ggtgccaagc cctcccagtg cggcctgtgt ccaagttacc tcacaccgaa 1200

tgtctaccct aagctgcacc acaggctgcg ggctcctcag ctccgtggcc tgacccacac 1260

ccacctacct ctcgcctctc tcacctgcct tgcccagtgg caccaaggag cgctagatct 1320

tgtggagaag aagatgggct ggactgtgga caaggtgtcc tcggaatcca agggaaagca 1380

ggggtag 1387

<210> 36
<211> 1325
<212> DNA
<213> Mus musculus

<400> 36
   ttgctacaag atgaaagccc tggatggttt aagagagtcc ctgtacccca gtctggactt 60
   ccagctctat caagatgacc aggtctgctc agctgacgct tctcagccac ttgcagactc 120
   agtgggtgct catgacttag cctggagcga gaggatgtgc cccttaccac tggcaccagc 180
   taaatctccc ttgctggcct gccccgaaag cccggaccta tgcttgtgtg ccctgcagaa 240
   gacaccccta ggcagagccc cacaggacct cggagaagac gcctcaaaca tgagacacca 300
   gccgccaagc ttgtacaaag cctccaccga cagtgagaaa ctcacaatca aggactcact 360
   gaacagagag gagatgggaa atgagccaga aagaggcgcc tacccacacc tccctccccg 420
   tactagctcc ttcccagatg ctgggctgga cagaaagagc cttagtccct tgaccttctg 480
   gccctggctt cctcccactc tcatctccaa ggaaccccct atccacatct accccatctt 540
   ccctgggtac ccacttctgc ctctccctta cctattcact tatggggccc taccttctgc 600
   ccaacatcca tacctcttca tgctgccccc acactccaca taccccaccg tggctgggcc 660
   cagcttgctt atgacagcca gtgggtctgg acctcgtatt ccccaagaga agaccctgct 720
   tctccactcg ggagctttcc agagtgccgg ccacacccta cactcccagg ttgagagtcg 780
   gagctctaga gatacccgga ccccaggaca agctggtgtg gcagctccaa caaggcgggc 840
   tgtgccaggc tcccgggcag gtgtcatagc cctgccctac cctctgaaga aagagaatgg 900
   ttcacctgcg agtacacagt ggcgagcgcc cgttccagtg tgccctgtgt cagaagcgct 960
   tcacccagct ggcccacctg caaaagcacc acctggtgca caccggggag cggccccacc 1020
   agtgccaggt gtgccacaag cgcttcagta gctccagcaa cctcaagacc catcttcggc 1080
   tgcactcagg tgccaagccc tcccagtgcg gcctgtgtcc aagttacctc acaccgaatg 1140
   tctaccctaa gctgcaccac aggctgcggg ctcctcagct ccgtggcctg acccacaccc 1200
   acctacctct cgcctctctc acctgccttg cccagtggca ccaaggagcg ctagatcttg 1260
   tggagaagaa gatgggctgg actgtggaca aggtgtcctc ggaatccaag ggaaagcagg 1320
   ggtag 1325

<210> 37
<211> 11141
<212> DNA
<213> Homo sapiens

<400> 37
ggggaggtag cacaaaggga ggaccatggt cttctaactg aggaacctga gcagatgcct 60

tgacccagac cagagaccca agccaaggaa agcccatgat caccagagta ggttgggaag 120

tgcggataga ggggaagtag tttttatttt tttctaacag agaagttgtg caaggtgttc 180

tgagagttgg gagcagagtg tgatgccagg agagggcaga gctggcagat ggaagccaaa 240

ggtgtgcggg tcaccccttc tgggtgggga catctgatcg acctagtcca tgtatgcctg 300

catgaagtgc tggtgaggga ggttggaatt aagtatttgg ccatttcggc ttttataatg 360

ggaggtacct tttgcctccc tcaagactca tgtaggggaa aagtttcaga ggctgggcgt 420

ccaaaaaaac ctgacagatg tctacatgga aaaccaaaat gagtagacag aggaccctgt 480

ccttgagatg ctcacaaaca tgttgggatc agttatgtgg aggatgagtc acagccctag 540
gggctgagct ggcagagaaa ccccaggaac atgccccagc ccatggtgca cagcctgaga 600
tgtccctgta tattctggga caccagggtg gcgtggctgg ggtgcagatt gatggctggt 660
tggggctttt gcaccctggg ccagactcac gcacaccaga atttgtgtaa ggccgcaggg 720
gtgatgcctc ttctcatgct cacaagaata atgacattgc tcaggctaaa aagccacttt 780
ctggcctatg aggtctccct ttttgtatct agacatgtct tttccttatt tattaacttt 840
ttttaagaca gggtctcgct ctgtcaccca ggctggagtg cagtggcatg atctcagctc 900
actgcagtct cgacctccct ggctcaagtg atcctcccac ctcagcctcc tgagtagctg 960
gaacaacagg catgcgctac catgcctggc taatttttgt attttttgta gagatggtgt 1020
ttcaccatgt tgctcatgct ggtcttgaac tcctgggctc aagcagttgg cccacctcag 1080
cctcccaaag tgctgggatt acagatgtga gccaccacac ctggcctagt catgccttac 1140
acccattttg ctatgtctat tttttttttt tttaaagaga cagggtcttt ctctgtcacc 1200
caggctagag tgcagtggca atatcctagt tcactgcagc cttatattcc tgggctcaag 1260
tgatcctcct gcctcagcct cctgagtagc tgggaccaga ggtgcaagtc accatgccca 1320
gctaagtttt gtattttttg tagagatgag gtcttgcttt gttgcccagg ctgatctcaa 1380
actcctgggc tcaagagatg ggtctgcctt ggtctcctga agtgctggga ttacagatgt 1440
gagccacttc acctggccat gtgcttgaat tatcttttgc ctcttggggc ctctagatta 1500
gaggatagaa ctttccaggg tcaggagatc tgagatggga cttggaggag gaaaacgtgt 1560
ttgctgtatc tctgtgagat cagctgcctt ctctgtcctc tgtctcattc catatccttc 1620
tatgttgcct gttcttgtct ctgcctgttc ctgtccctac ctgtcaccat cactgtctcc 1680
ctgtcatcat caatgcctat cttcatctca gcttggctcc cctccacaga aggctcccct 1740
gttcagcctt tgagccctac tctgtcctcc cgtccagcca tctgggtact ttgagaacag 1800
aaataacagc ccgcccccct cacacacaca tcctgaaatt ccagtagccc cagttcactg 1860
caatccacat cagcaggttg tcctccagag ttgccacagg taggcaaagc tgagtggata 1920
gtgccccctt gtcagacctt gcagggcccc caagcctgcc tgcccccact tcctatctgt 1980
ggcaacttca aatgtaacac cttcctgcca tctctcactc ccactggggc ctcagccaca 2040
ggcagctgtg gcctggctgc ccacctcctt ccttcccagc caggagcact gctgcagcac 2100
cgagtggaga gccccacagc cagcagccct tcgggggtcc atggtatttg ctttgagacc 2160
tgaagcagcg ttgggaggag tgaggaaaga gggaaaggag aaggggtttt agagggctga 2220
gacactggag tgagggtctt agggtctgag gggcatttgg gattcaaaga gtggtccctg 2280

aggtgacggg gaaggggtct tggggcattt gctgggctga agtgttccct gtgctgtgtg 2340

gcctcaggtg gaagatggca gagaagcctg gtgctggatg agcagacagc tgggcactcg 2400

cgtctctctg tcaggccagt caggggcatc cgggggcttg gtccactcat tcaggcttta 2460

ttcctggtga gagcgctgag ctgggcagcc tgggtgacac gacactgagg tgtcctcagc 2520

tgtccagaag agctatctgg gtgagctagg atgcaggatt ccccttggtg gggggcctct 2580

ggttggtaga gtcagggacc cctctcagca cagaaggggg tggcttcttt gtagccctga 2640

ggaatctgga atgttccctc agatctctgc acttacttgg aagtaagctc cgtgagggcc 2700

ttcctctcct ctcaggagag ctctgctctt cacttctgat tggctaatct ccattgattg 2760

attgattgat tgattgattg agacagggtc ttgctctatc ctcgcccagg ctagagtgta 2820

gtggtgtgat ctcagctcac tgcagccaca accttccagg ctcaagcaat cttcccacct 2880

cagcctccca agtagctggg accacaggca caggctacta cacccagcta atttatttat 2940

ttatttttga accgagtctc actctgttgc tcagactgga gtgcagtgac atgatctcgg 3000

ctactgcaac ctccgcctct tgggttcaag cgattctctt gcctcagcct ccctagtagc 3060

tgggactaca ggtgtgagcc accacactca gctaattttt tttttttgga gacaaagtct 3120

tgctctgtca cccaggctag aatgcagtgg tgctatcttg gctcactgca acctctgcct 3180

cccgggttca agcgattctc ctgcctcagc ctcctgagta gctgggatta caggcgtgcg 3240

ccaccacacc cggctaattt atgtagtttt ggtagagacg aggtttcacc atgttcgtca 3300

ggctggtctt gaactcccaa cctcaggtga tccgcccgct tcagcctccc aaagtgctgg 3360

gattacaggc atgagccacc gcacctggtc taattttaat ttttggattt ttagtagaga 3420

tgggggtttc accatgttag ccaggctggt ctcgaactcc tgacctcagg tgatccatct 3480

gcctgggtct cccttttatt ttttattttt ttatttattt tattttattt ttagaaggag 3540

tctcattctg ttgcccaggc ttgaatgcag tggcacgatc ttggctcact gcaacctcca 3600

cctcccaggt tcaagtgatt cttgtgcctt ggcctcctga gtagctggga ttataggcat 3660

gcaccaccgt gactggctaa tttttgtatt tttagtagag acagggtttc accatgttgc 3720

cgaggttagt cttgaactcc tgacatcagg tcatccaacc gccttggact cccaaagtgc 3780

tgggattaca ggcctgagcc accccgccca ccatattttt tattttgtag agatgggggt 3840

ctcactatgt tgcccaggct ggtctagaac tcctggcctc aaaagatcct tccttctcag 3900

cctcccaaag tgctgggatt acaggcttga gccacggcac ctggccaatc tctatttaat 3960

cttcccacac ccaccaggag agcatcactg gcttctaaat gtcaacattg agagaatcct 4020

tacgcatcaa ctagaccaac cttcccattt tgcggatagg gaaactgagt ccaggaaggg 4080

acatgaataa cttgtggtca tgcagcatgt taatgggaga gccagtccta gaactcagac 4140

tcctatttgg aggaccttca cagcttacct ctaactcacc tgtttcccag caggtaatgg 4200
ggatatgaag gaagaatcag ctgcacaatt aggttgttgt cataggccca tggccctggg 4260
aggtacaggg ggctccctgt cccccagcct ggacttccag ctcttccgag gtgaccaggt 4320
aaggtagaac cccagcctta cctttgtggg tagtctgctt atagtaggag gtgacattag 4380
gaggaaaaag gggaacgtct gaagggacct agatctcctt cccagtctga gcacccccta 4440
tcccaggcac atcataggga tgtttctttg aggagccaga caccctctcc ccatgcctca 4500
ggggcccaga ctctcctgtg gggaataaga agcggaagcc tcgtgcagta ggcttggctg 4560
ctatgaccat ggccttgggc cttagtgagc aggggctcag ggccatcttg tggagtttat 4620
agtctttgat ggggattttc tttgtgctca acttggtgcc attcaaatta cagtgatggt 4680
ctgagataca ctggggacca gggtggtcag aggtacatgg gttcggggca gggagggaga 4740
acagggctag agagggccct ggggacagga ggagggcaat cttgtggtgg gtgggaaaat 4800
cctgggggaa agagatcttt ggatgaggga agagatgcca tttgagtgac agatatggag 4860
tgtggtggct gttttagctt attctcccct ttggaaaaca aaataggggt aggggccatc 4920
taagggaccc atggagtccc agtcaccagc cccaagcccc cttgcccctg ccctccaggt 4980
cttctcagcc tgcagaccac ttccagacat ggtggatgct catggcccat cctgtgccag 5040
ctggctgtgt cccttgcccc tggcaccggg caggtctgca ctgctggcct gcctacagga 5100
cctggacctg aacctgtgca ccccacagcc ggcacccctg ggcacagacc tgcagggcct 5160
ccaagaggac gccttgagca tgagtaagcc cagggcaccg ggctgcacct gcgcctggca 5220
gaggctggag ggtggggggc actgctaggt ttggggatgg gtggtggaga ggacgcagtg 5280
tcactgatga agacagagct taggcacact ttacttccac attttggcct tggagttctg 5340
gcctgagggt gcagggggct ttcgagtggt gatacctaag ccagagaagc tgggcactgg 5400
cttctcaggc atgtgtgtgg gagctgatgt aggtatagcc aaggtcatgg gggttgggga 5460
tggtgctttg tctgcttaga gaggtgtttt ttgtttgttt gtttgttttt gagatggagt 5520
ctcgttctgt tgcccaggct ggagtgcaat ggcatgatct ccagtcaccg caacctctgc 5580
ctcccaggtt caagcgattc tcctgcctca gcctctcgga gtagctggga ttacaggtgt 5640
gcgccaccac ccccggctaa tttttgtatt tttagtaaag acagggtttt gccatgttgg 5700
ccaggctggt ctcgaactcc tgacctcagg tgatccacct gcctcggtga cccaaagtgc 5760
tggaattata gatgtgagcc actgtgccca gccctagagg gatcttataa aagtactgga 5820
ggccgggcac ggtggctcat gcctgtaatc ccagcacttt gggaggctga ggcgggcaca 5880
tcacgaggtc aggagatcga gaccatcctg gctaacacgg tgaaacccca tctctactaa 5940

aaatacaaaa aattagctgg ggggtggtgg caggtgcctg tagtcccagc gactcaggag 6000
gctgaggcag gagaatggca tgaacctggg aggcggagct tgcagtgagc cgagatggca 6060
ccactgcact cagcctgggc gactgagcga gactccatct caaaaaaaaa gcactgggct 6120
gggagtcagg gaattggggt tggagtctcg gttctgccct ttctatttct atggacttgg 6180
acacaacacc aaggtctggt ttcttcatcc ataaaagtgt gatctcatct ctgcctgggt 6240
tctaggtttg ttgtaacaca ccatttcctt atgtgacgat gaccccctga aggcaaggac 6300
tctcttgctg tgtcctttat ttctaggacc ccagcacaga gcacagagtg aaacggctca 6360
gtgagattga ttgagtaaat aaataaggat ccaaccagaa gacaacttgc tttttttttt 6420
tttttttttt tttgagatag gctggagtgc agggacacaa tcatggctca ctgcagcctc 6480
caactcctgg gctcaagcaa tcctctctcc tcagcctcct gggtagctgg gactactaca 6540
gacacatgcc acaaacaccc agctaatttt ctttctttct ttctttcttt tttttgaaac 6600
agagtttcgc ttttgttgcc caggctggag tgcaatggca tgatctcggc tcaccgcaat 6660
ctctgcctcc cgggttcaag tgaatcttct gcctcagcct cccaagtagc tgggattaca 6720
ggcatgtgcc accatgccca gctaattttg cctttttagt agagacgggg tttctccatg 6780
ttggtcaggc tggtcttgaa ctcccaacct caggtgatct tcccgcctcg gcctcccaaa 6840
gtgctgggat tacaggcgtg agccaccgtg cccagcctta attttcttat tgttttgtag 6900
agatgggatt ttgctttgtt gcccaggctg gtctcaaatt cctaggctta agcaatcctc 6960
ctgcctcagc ctcccaaagc actgggatta taggcatgag ccactgcacc tggctgacag 7020
tttatttttt aacaggtttt ttattttagt actctttatc tagaatagca ctgtttggta 7080
gaactccctg caataatggg aacattctat acctgtgctg tccaatacta tacctgctga 7140
gcactgtaaa tgtgactggt gcaactgaga acttcatttt ttattagaat cattgtaatt 7200
aatgtaaatc tagtcatgca tccagtggct gccatttcgg acatagcaga tctagcctca 7260
cccttatgtg atcttatctg tgcccatggc acctggattt atgtctccag ttcagacctc 7320
tcctttaaac tccaacccag tttatccatc tgcccacttg gcttctcaac ctgggtgtct 7380
aatagggacc ttgaactgct ttctccccct gaaattgttc agcactacag aaatgctagc 7440
aggctcgcag gctggaggtc tttctcagcc ccgagcaggg caggtttgat ggcggacaga 7500
acttcacccc agactgtggg gatctgaccg ccttcctttt cctgcttaga gcacgagcca 7560
ccagggctgc aggccagctc caccgatgac aagaaattca cagtcaagta cccacagaac 7620
aaggacaagc tgggaaaaca gccagaaaga gctggcgagg gggccccctg cccagccttc 7680
tcctctcata acagctcttc cccaccaccg ctgcagaaca gaaagagccc cagccccttg 7740
gctttctgcc cctgtccccc tgtcaactcc atctccaagg agctcccatt tctcctccac 7800

gccttctacc ctggataccc acttctcctg cctccacccc acctgttcac ctatggggcc 7860
ctaccttctg accaatgtcc ccacctcctc atgctgcccc aagacccctc ctaccccacc 7920
atggctatgc ctagcctgct gatgatggtc aatgagctgg ggcaccccag cgctcggtgg 7980
gagaccctgc ttccctaccc aggggccttc caagcctctg gccaagctct gccttcccag 8040
gcccgaaatc caggtgctgg agctgcccca accgactccc caggcctgga gcgtggtggc 8100
atggcatctc cagcaaagcg ggtcccattg agttcccaga caggcaccgc agccttgcct 8160
tacccgctga aaaagaagaa tggcaaaatc ctgtacgagt gcaacatatg tggcaagagc 8220
tttgggcagc tctccaatct caaggtatct gcctcctggg ctctgcttct cccctcaaca 8280
tccttcaggg ccccccatgc acccgagtct gcttctagaa gcaaggacct caaagctaga 8340
aggcactcct gggcaacctc tagggccaag tgaccttgga tggttcattt gggtggagat 8400
atcggataag acggccacca aagtcctcgc caccctagag gactgcctat gagatcgtct 8460
catttaggtt aaaatgggga gactgaggct tttaatgggc agcgtttgcc taagattacc 8520
ccgatttaac ggtagtgtta ggtttagtct ctcaacattt gctctgggca aagaaagccc 8580
ttacctggac aaccatcctt tctggactcc aagttaagct tcttatttat ttttttgggc 8640
agtcagatga gggaatgggt agattttggt gagtctagac cacagtccga tgaccaacct 8700
ttttcaagtg ggatcccaca aatctgcgcg accgccgatg cgattggcct caccatccgc 8760
atcgcccagc agggggcgcc cagggagcgc atctcagtgc gttagcaaag ggcggaaact 8820
gtgcgctctc tggctagttc tgaagttgaa ggcacattgt ggttatgggg agaagccctg 8880
ctggatcctg cagggcagga gttcttaaac tttcatgagc ctcagaccac ctggagagct 8940
agtaaacatg cagattccta gagtttttgt gatcctccca cctcagcctc cagagtagct 9000
cccagcccca gagttatctt tttttttttt ttttttttcc atatggagtc tcattctgtc 9060
gcccacgctg gtctcaaact cctgggctta agggattctc ccacttcatc ctccccggta 9120
gctgggacga agggtaaaca ccaccactcc tcgttaactt tcaaattttt tgtgcatacg 9180
gggtcccaat gtgtttctca ggctggtatt gaactcctgg gctccattga tcctccctcc 9240
tcggcctccc aaaatgctgg gattacagcc acgagagtgt gcccagccct cccagagttt 9300
ttgattcagc aggtctgggg ccggacttga gaacctgcat ttctaacatg ctcccaaagt 9360
gtgataccag cactgctggt ctgtgaacca cattttgaat agcagtgttc cacaatatac 9420
ctggggtacc tcttacccct ggccttctga ccactgccat ctcccctaca gggcagcaga 9480
aagagtagag tatcctgggg atgatgggcc ctgagggaga tgacggggag ggagagagaa 9540
gtgggcagag atttggagag gacctcaggc cctgctaggg gtgctgggca gttggcagca 9600

tttatcccgc atctgactca ggtccacctg cgtgtgcaca gtggagagcg tccattccag 9660

tgtgccttgt gccagaagag cttcactcaa cttgcccacc tgcagaagca ccacctggtg 9720

cacactgggg agcggcccca caagtgctcg gtgagatcct tctcccccta ctatgtactc 9780

ctataggctg gagaggagtt ttaggccctt ggagggctgg cacctgcagc ctccattccc 9840

cagtcctcct gagcaagtgg agatgggaag ggggagtgaa aattgaaaat gactcctctc 9900

agcctggaat gggtaatact gaaggtcact ccaaaggcct gcaggaggga gagaacaatg 9960

aagttggagg tcatggagag tgtatgtata cccattatct acctccctga gttattggag 10020

gagttaatgc tatagtgtac ccagagtatc caagtaggaa aactattagc ttgatgtcag 10080

ctccctcctt ctgtgagcct ggccacatcc ctcgggaaat cagaagggca ggggttcttt 10140

cccattttac aggggcagaa atgaaagcag tgccatgatt atgagtccgg gctgcatagt 10200

cgggccacct gtgttcaaat cctagctctg ccttcaactt gctgtgtgac ctttggctag 10260

tggcttcact cctctgagac tctgctttct tactataata taggacttgt actaatactt 10320

agtcatgctt ccctagggtc ctggggaaga ctagatgaaa ccaactgcag gcatgtggca 10380

gccccctgta aatgttcatt acaatctact gaagctgcag cttcatactt aagagttcta 10440

tgtgattatc tcattcagtc caccatatct atgaagctag gtattaataa ccaatgttac 10500

caatgaggag aggaaggctc agagaggaga agttactttt acaaggtcac acatggagta 10560

aaggacacta acccaagttg gcctggttct aatgtgctag ataccgtggg ttccaggaag 10620

aaatcactgg aaaagcttcc aggcctggag ggaaagagag gtgggtcatg ggtatttcca 10680

agatgaggag agggagtaga atgcctgagc cagcccagag gacaaagctg atgccagtgc 10740

tcgttcccgt caggtgtgcc acaagcgctt cagcagctcc agtaacctca agacccacct 10800

gcgcctgcac tccgggcccc ggcccttcca gtgcagtgtc tgccggagtc gcttcaccca 10860

gcacatccac ctgaagctgc accatcggct gcatgcccca cagccctgtg gcctggtgca 10920

cacccagctg cccctggcct ctctggcctg ccttgcccaa tggcaccagg gggcactaga 10980

tcttatggcg gtggcatctg agaaacacat gggctatgac atagatgagg tcaaagtgtc 11040

ctcgacatcc caggggaaag caagagcagt gagcctgagc agtgccggga ctcccctggt 11100

gatggggcag gaccagaaca attaaaaatg tttcttctgt c 11141